# EUROPEAN PATENT APPLICATION

(11) **EP 1 882 705 A1**
(43) Date of publication of application: **30.01.2008**
(21) Application number: 06746393.5
(22) Date of filing: 15.05.2006
(51) Int. Cl.: C08F 20/28, C08F 20/54, C08F 32/08, C08F 38/00, G03F 7/039

(54) **POLYMERIZABLE COMPOUND FOR PHOTORESIST, POLYMER THEREOF, AND PHOTORESIST COMPOSITION CONTAINING SUCH POLYMER**

(30) Priority: 20.05.2005 JP 2005148737
(71) Applicant: IDEMITSU KOSAN CO., LTD., Tokyo 100-8321 (JP)
(72) Inventor: HATAKEYAMA, Naoyoshi, Chiba 2990193 (JP); ITO, Katsuki, Chiba 2990193 (JP); ONO, Hidetoshi, Chiba 2990193 (JP); MATSUMOTO, Nobuaki, Chiba 2990193 (JP)
(74) Representative: Vossius & Partner
(86) International application number: PCT/JP2006/309646
(87) International publication number: WO 2006/123605

(57) **Abstract**

The present invention provides a polymerizable compound having an alicyclic structure and a polymerizable group and represented by the general formula (1) or (19). The polymerizable compound exhibits high adhesion to substrates, reduced swelling during development, a reduced amount of water absorption during exposure in water, and high dry-etching resistance. Also provided are a polymer of such a polymerizable compound, a process for producing the same and a photoresist composition containing the polymer.

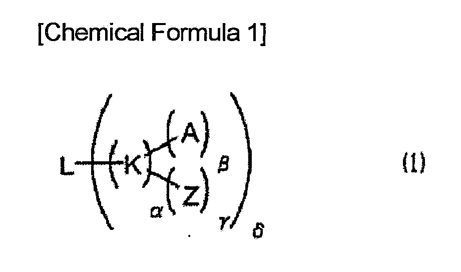

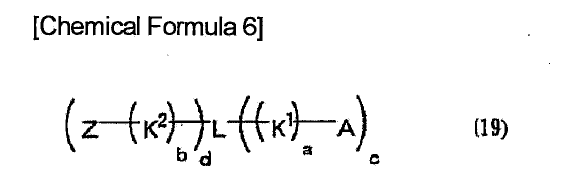

## Description

### Technical Field

The present invention relates to a polymerizable compound for a photoresist, to a polymer thereof, to a process for producing the polymerizable compound and to a photoresist composition containing the polymer. More specifically, the present invention is directed to a polymerizable compound for a photoresist used in the field of a photoresist lithography, to a polymer thereof, to a process for efficiently producing the polymerizable compound and to a photoresist composition containing the polymer.

### Background Art

An adamantane structure in which four cyclohexane rings are condensed to form a cage-like structure is stable and highly symmetrical. It is known that adamantane derivatives, which show peculiar functions, are useful as raw materials for medical materials, raw materials for highly functional industrial materials, etc.
For example, because of good optical characteristics and heat resistance of adamantane derivatives, an attempt has been made to use them as photo disc substrates, optical fibers, lenses, etc. (for example, Patent Document 1 (JP-A-H06-305044) and Patent Document 2 (JP-A-H09-302077)).
Further, because of sensitivity to acid, dry-etching resistance and transparency to UV rays of the adamantane esters, an attempt has been also made to use them as raw materials for resins used for photoresists (Patent Document 3).

On the other hand, as semiconductor elements are made finer in recent years, a lithography step in the production thereof is required to form more finer patterns. In this circumstance, various methods have been examined for the formation of fine patterns using photoresist materials suitable for irradiation with a beam having a short wavelength such as a KrF, ArF or F₂ excimer laser beam.
For the purpose of improving adhesion of a photoresist to substrates, hydrophilic sites are incorporated thereinto. The presence of such hydrophilic sites, however, is known to cause a problem of the swelling of patterns after development.
An exposure technique has now developed into liquid immersion lithography. Here, too, the presence of the above-described hydrophilic sites causes a problem of water absorption of the photoresist resin.
For example, a photosensitive composition using a compound having a tricyclodecanyl structure, which is a bridged alicyclic compound, is known (Patent Document 4). When a resist pattern is formed from a resist of such a composition by alkali development, however, various problems arise because of a significant difference in solubility in the alkali between the alicyclic structure, such as an adamantane structure or a tricyclodecanyl structure, having high hydrophobicity and a soluble group such as a carboxylic acid group.
Thus, such a difference causes non-uniformity of dissolution and removal of intended region of the resist film at the time of development and, therefore, a reduction of resolution. The reduction of resolution also results from the swelling of the resist pattern after the development. Further, partial dissolution of a region of the resist film which should remain undissolved causes cracks and surface roughness.
Additionally, delamination of a resist pattern is often caused because of penetration of the alkaline solution into the boundary between the resist film and the substrate.
Phase separation between the region having an alicyclic structure and the region of the soluble groups, such as carboxylic acid groups, of the polymer is apt to proceed. This will cause a difficulty in preparation of homogeneous resist coating liquid as well as insufficient coatability.
Thus, there is a demand for a photoresist material or a monomer thereof capable of reducing swelling of fine patterns.

Patent Document 1: JP-A-H06-305044
Patent Document 2: JP-A-H09-302077
Patent Document 3: JP-A-H04-39665
Patent Document 4: JP-A-H07-199467

### Disclosure of the Invention

The present invention has been made in view of the above circumstance and has an object to provide a polymerizable compound for a photoresist capable of exhibiting high adhesion to substrates, reduced swelling at the time of development, a reduced amount of water absorption during exposure in water, and high dry-etching resistance, and, further, to provide a polymer of such a polymerizable compound, a process for producing such a polymerizable compound and a photoresist composition containing the polymer.

With a view toward solving the above problems, the present inventors have made an intensive exploration of compounds which permit elimination or deprotection with an acid, whose alicyclic structure can remain present after alkali development and, further, compounds which have a hydrophilic group, such as a lactone group exhibiting adhesion to substrates, linked to an alicyclic structure. As a result, a polymerizable compound having an alicyclic structure and a terminal group has been found useful as a resist of a photoresist composition and thus the present invention has been completed.
The present invention has been completed on the basis of the above findings.

Namely, in accordance with the present invention there are provided:
1. A polymerizable compound having an alicyclic structure and a polymerizable group and represented by the general formula (1): 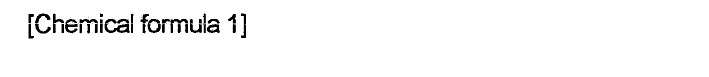 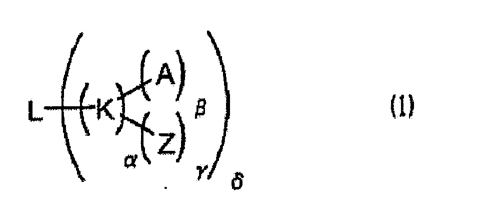 wherein
   A represents the polymerizable group selected from those represented by the following formulas (2), (3) and (4): 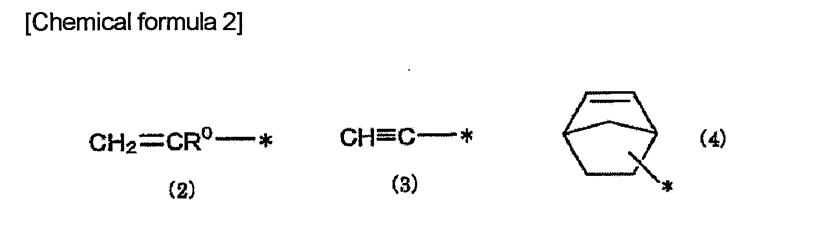 (wherein R⁰ represents a hydrogen atom, a fluorine atom, a methyl group, an ethyl group or a trifluoromethyl group)
      with the proviso that when there are a plurality of such A groups, they may be the same or different;
   K represents a linking group selected from those represented by the following formulas (5), (6), (7), (8) and (9): 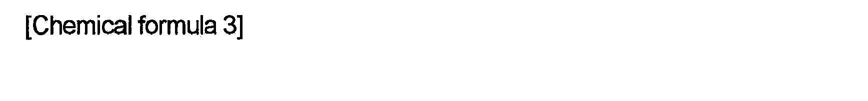 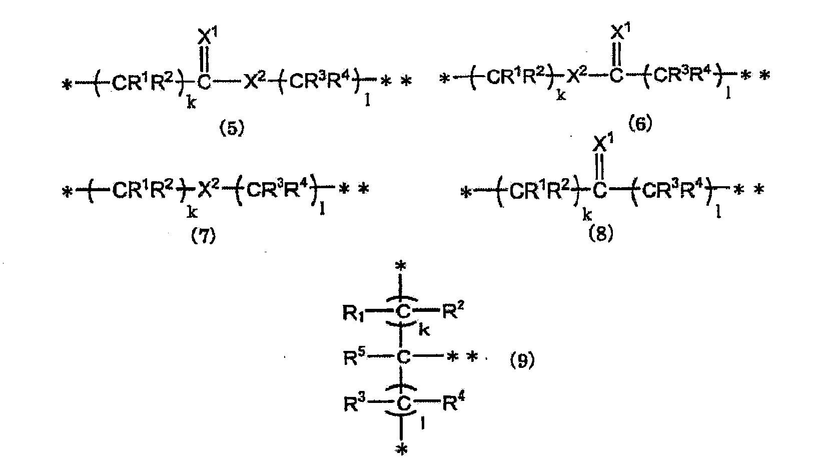 (wherein R¹, R², R³, R⁴ and R⁵ each represent a hydrogen atom, a C₁ to C₁₀ alkyl group which may contain a hetero atom, or a halogen atom, k and I each represent an integer of 0 to 10, X¹ and X² each represent an oxygen atom, a sulfur atom or an NH group, * indicates the bond to the polymerizable group or a terminal group, and ** indicates the bond to an alicyclic group) with the proviso that when there are a plurality of such K groups, they may be the same or different;
   L represents the alicyclic group selected from those represented by the following general formulas (10), (11), (12), (13), (14), (15) and (16): 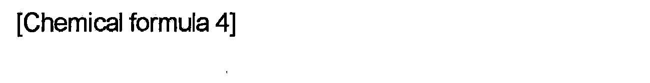 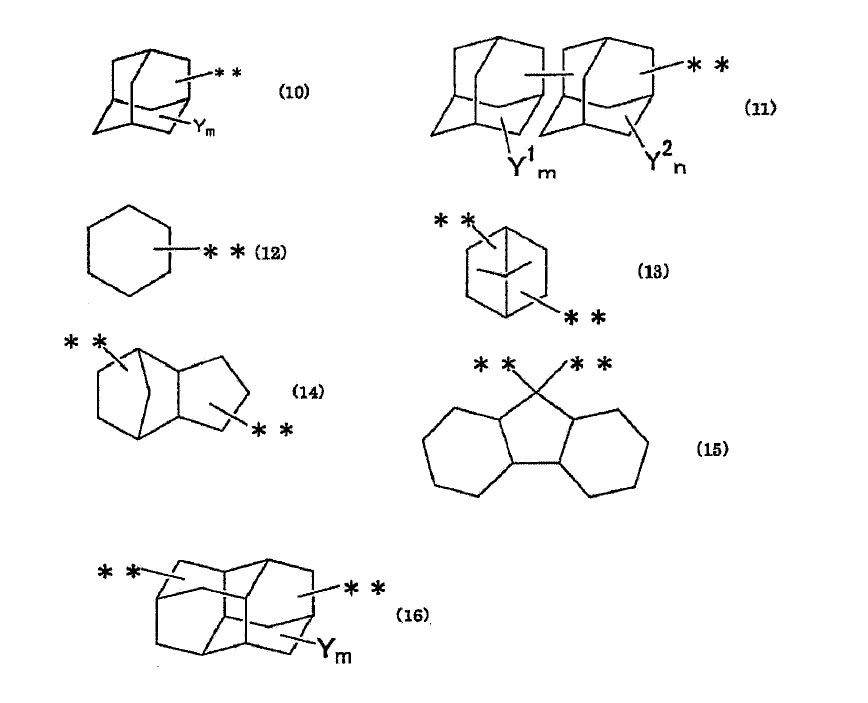 (wherein Y Y¹ and Y² each represent a hydrogen atom, a C₁ to C₁₀ alkyl group, a halogen atom, a hydroxyl group, a mercapto group or a methylcyano group, or two Ys, two Y¹s and two Y²s are each taken together to represent =O or =S, with the proviso that when there are a plurality of such Y groups, a plurality of such Y¹ groups and a plurality of such Y² groups, they may be the same or different, respectively, and m and n each represent an integer of 0 to 15);
   Z represents the terminal group selected from those represented by the following formulas (17) and (18): 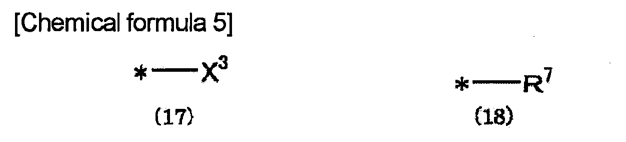 (wherein R⁷ represents a C₁ to C₃₀ alkyl group or a C₅ to C₃₀ cycloalkyl group each of which may contain a hetero atom, a hydroxyl group, a mercapto group, an ether group, a thioether group, a cyano group, a ketone group, a thioketone group, a ketal group, a thioketal group, an acetal group, a thioacetal group, a lactone group, a thiolactone group, a carbonate group, a thiocarbonate group, an amine group, an amide group, an alkylsulfonyl group, an ester group or a thioester group, and X³ represents a hydrogen atom, a hydroxyl group, a mercapto group, an amino group, PO₃, a nitro group, OSO₂CH₃, SSO₂CH₃ or NHSO₂CH₃)
      with the proviso that when there are a plurality of such Z groups, they may be the same or different; and
   a is an integer of 1 or more, β and γ are each an integer of 0 or more and δ is an integer of 1 to 16, with the proviso that the polymerizable compound contains at least one A group and at least one Z group;
2. The polymerizable compound as described in 1 above, wherein L in the general formula (1) is a group represented by the formula (10) or (11);
3. The polymerizable compound as described in 1 above, wherein at least one of K in the general formula (1) is a group selected from those represented by the formula (5) and (7);
4. A polymerizable compound having an alicyclic structure, a polymerizable group and a terminal group and represented by the general formula (19): 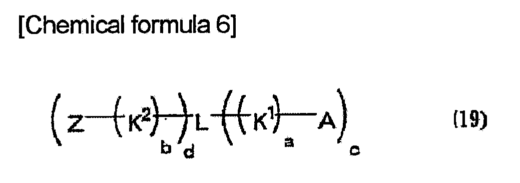 wherein
   A represents the polymerizable group selected from those represented by the formulas (2), (3) and (4): 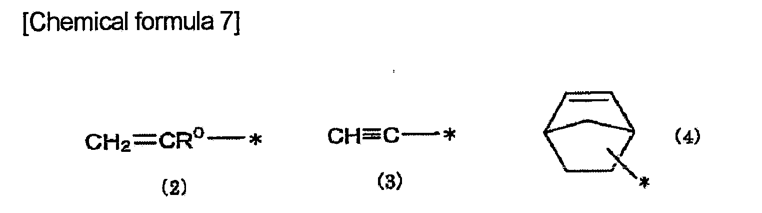 (wherein R⁰ represents a hydrogen atom, a fluorine atom, a methyl group, an ethyl group or a trifluoromethyl group)
      with the proviso that when there are a plurality of such A groups, they may be the same or different;
   K¹ and K² each represent a linking group selected from those represented by the following formulas (5), (6), (7), (8) and (9): 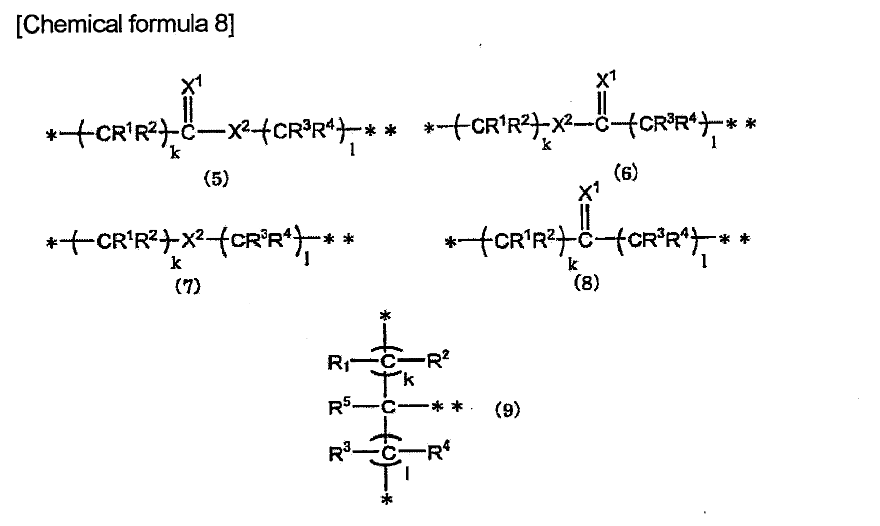 (wherein R¹, R², R³, R⁴ and R⁵ each represent a hydrogen atom, a C₁ to C₁₀ alkyl group which may contain a hetero atom, or a halogen atom, k and I each represent an integer of 0 to 10, X¹ and X² each represent an oxygen atom, a sulfur atom or an NH group, * indicates the bond to the polymerizable group or the terminal group, and ** indicates the bond to an alicyclic group)
      with the proviso that when there are a plurality of such K¹ and K² groups, they may be the same or different;
   L represents the alicyclic group selected from those represented by the following general formulas (10), (11), (12), (13), (14), (15) and (16): 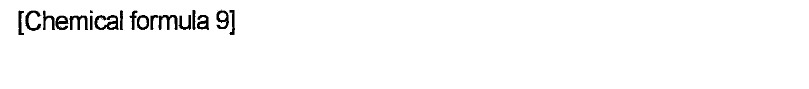 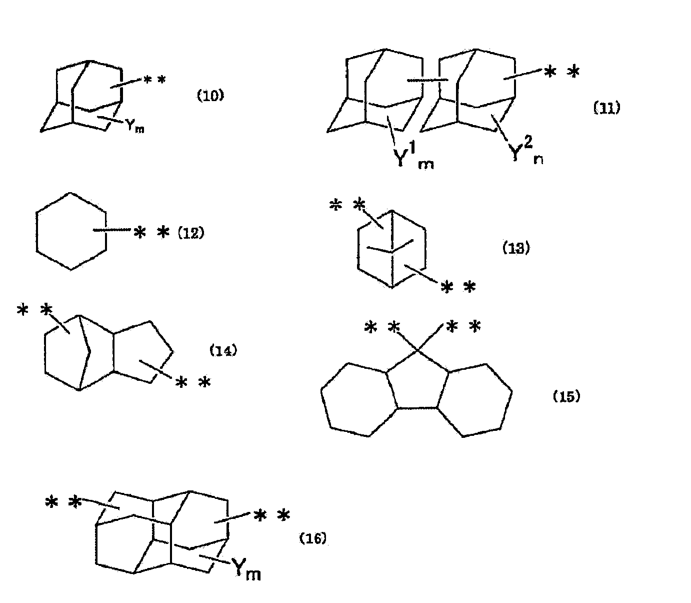 (wherein Y, Y¹ and Y² each represent a hydrogen atom, a C₁ to C₁₀ alkyl group, a halogen atom, a hydroxyl group, a mercapto group or a methylcyano group, or two Ys, two Y¹s and two Y²s are each taken together to represent =O or =S, with the proviso that when there are a plurality of such Y groups, a plurality of such Y¹ groups and a plurality of such Y² groups, they may be the same or different, respectively, and m and n each represent an integer of 0 to 15);
   Z represents the terminal group selected from those represented by the following formulas (17) and (18): 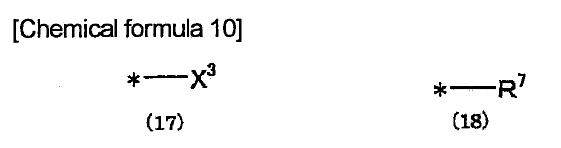 (wherein R⁷ represents a C₁ to C₃₀ alkyl group or a C₅ to C₃₀ cycloalkyl group each of which may contain a hetero atom, a hydroxyl group, a mercapto group, an ether group, a thioether group, a cyano group, a ketone group, a thioketone group, a ketal group, a thioketal group, an acetal group, a thioacetal group, a lactone group, a thiolactone group, a carbonate group, a thiocarbonate group, an amine group, an amide group, an alkylsulfonyl group, an ester group or a thioester group, and X³ represents a hydrogen atom, a hydroxyl group, a mercapto group, an amino group, PO₃, a nitro group, OSO₂CH₃, SSO₂CH₃ or NHSO₂CH₃)
      with the proviso that when there are a plurality of such Z groups, they may be the same or different; and
      a and b are each an integer of 1 or more, c and d are each an integer of 1 to 15, with the proviso that c+d ≤ 16;
5. The polymerizable compound as described in 4 above, wherein, in the general formula (19), A is a group represented by the formula (2), and at least one K¹ is a group represented by the formula (5) with the proviso that k is 0;
6. The polymerizable compound as described in 4 above, wherein, in the general formula (19), A is a group represented by the formula (2), at least one K¹ is a group represented by the formula (5) with the proviso that k is 0, and at least one K² is a group represented by the formula (7) with the proviso that I is 0;
7. The polymerizable compound as described in 6 above, wherein, in the general formula (19), A is a group represented by the formula (2), at least one K¹ is a group represented by the formula (5) with the proviso that X¹ and X² are each an oxygen atom and that k is 0, and at least one K² is a group represented by the formula (7);
8. The polymerizable compound as described in 7 above, wherein, in the general formula (19), A is a group represented by the formula (2), at least one K¹ is a group represented by the formula (5) with the proviso that X¹ and X² are each an oxygen atom and that k is 0, and at least one K² is a group represented by the formula (7) with the proviso that X² is an oxygen atom and that I is 0;
9. The polymerizable compound as described in 4 above, wherein, in the general formula (19), A is a group represented by the formula (2), at least one K¹ is a group represented by the formula (5) with the proviso that k is 0, and Z is a terminal group having a thiolactone group or a thioester group;
10. The polymerizable compound as described in 4 above, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that l is 0, and Z is a terminal group having a thiolactone group or a thioester group;
11. The polymerizable compound as described in 10 above, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that I is 0, and Z is a terminal group having a thiolactone group or a thioester group;
12. The polymerizable compound as described in 4 above, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that I is 0, L is a group represented by the formula (10) or (11), and Z is a terminal group having a thiolactone group or a thioester group;
13. The polymerizable compound as described in 12 above, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that l is 0, L is a group represented by the formula (10), and Z is a terminal group having a thiolactone group or a thioester group;
14. The polymerizable compound as described in 4 above, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that I is 0, and Z is a terminal group having a lactone group or an ester group;
15. The polymerizable compound as described in 4 above, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that I is 0, L is a group represented by the formula (10) or (11), and Z is a terminal group having a lactone group or an ester group;
16. The polymerizable compound as described in 15 above, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that l is 0, L is a group represented by the formula (10), and Z is a terminal group having a lactone group or an ester group;
17. A process for producing a polymerizable compound represented by the general formula (19a) given below and having an alicyclic structure and a terminal group, comprising reacting a compound represented by the general formula (20) given below with a compound represented by the general formula (21) given below: 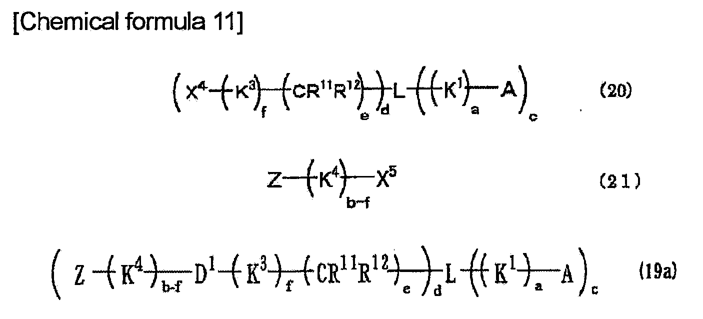 wherein
   X⁴ represents a group selected from a hydrogen atom, a halogen atom, an alkylsulfonyloxy group, a perfluoroalkylsulfonyloxy group and an alkyl-substituted phenylsulfonyloxy group, in which case X⁵ represents a group selected from a hydrogen atom, a hydroxyl group, a mercapto group and an NH₂ group, or X⁴ represents a group selected from a hydrogen atom, a hydroxyl group, a mercapto group and an NH₂ group, in which case X⁵ represents a group selected from a hydrogen atom, a halogen atom, an alkylsulfonyloxy group, a perfluoroalkylsulfonyloxy group and an alkyl-substituted phenylsulfonyloxy group;
   D¹ represents a linking group formed by the reaction between X⁴ and X⁵;
   K³ and K⁴ each represent a linking group;
   A, K¹, L, Z, a, b, c and d are the same as defined above; and
   e is an integer of 0 to 10 and f is an integer of 0 or more;
18. A process for producing a polymerizable compound represented by the general formula (19b) given below and having an alicyclic structure and a terminal group, comprising reacting a compound represented by the general formula (22) given below with a compound represented by the general formula (23) given below: 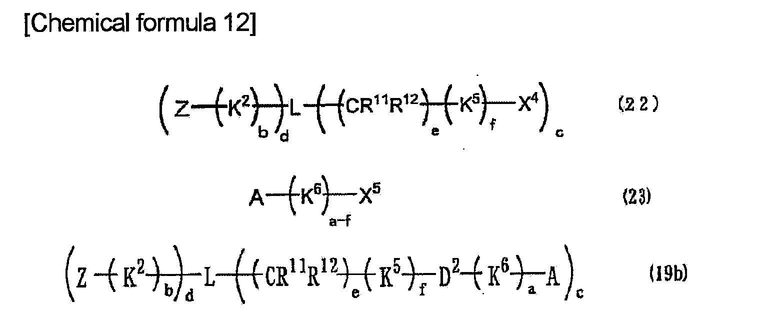 wherein
   X⁴ represents a group selected from a hydrogen atom, a halogen atom, an alkylsulfonyloxy group, a perfluoroalkylsulfonyloxy group and an alkyl-substituted phenylsulfonyloxy group, in which case X⁵ represents a group selected from a hydrogen atom, a hydroxyl group, a mercapto group and an NH₂ group, or X⁴ represents a group selected from a hydrogen atom, a hydroxyl group, a mercapto group and an NH₂ group, in which case X⁵ represents a group selected from a hydrogen atom, a halogen atom, an alkylsulfonyloxy group, a perfluoroalkylsulfonyloxy group and an alkyl-substituted phenylsulfonyloxy group;
   D² represents a linking group formed by the reaction between X⁴ and X⁵;
   K⁵ and K⁶ each represent a linking group;
   A, K², L, Z, a, b, c and d are the same as defined above; and
   e is an integer of 0 to 10 and f is an integer of 0 or more;
19. A polymer comprising, as a constituting component, a polymerizable compound having an alicyclic structure and a polymerizable group and represented by the general formula (1); and
20. A photoresist composition comprising a polymer comprising, as a constituting component, a polymerizable compound having an alicyclic structure and a polymerizable group and represented by the general formula (1).

The photoresist composition of the present invention, which comprises a polymer containing, as a constituting component, a polymerizable compound having an alicyclic structure and a terminal group, has high adhesion to substrates because of the presence of hydrophilic groups. Further, because of the presence of the highly hydrophobic alicyclic structure, the photoresist composition exhibits reduced swelling during development and a reduced amount of absorbed water at the time of exposure in water.
In particular, when the alicyclic structure has an adamantyl group, the photoresist composition exhibits high dry-etching resistance and excellent performace.

### Best Mode for Carrying Out the Invention

The present invention pertains to a polymerizable compound having at least an alicyclic structure and a polymerizable group and represented by the general formula (1) or general formula (19).
Examples of the polymerizable compound having a plurality of linking groups are as follows:

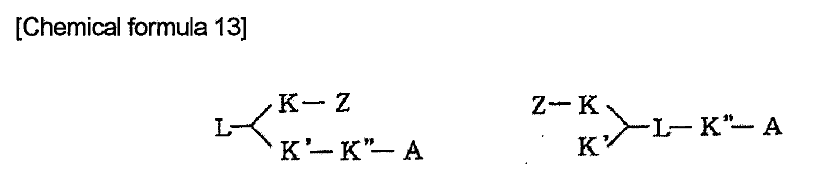

Z-K^{2'}-K²-L-K¹-K^{1'}-A

In the above formulas (5), (6), (7), (8) and (9) showing the linking groups (K, K¹ and K²), examples of the C₁ to C₁₀ alkyl group, which is represented by R¹, R² R³, R⁴ and R⁵ and which may contain a hetero atom, include a methyl group, an ethyl group, an n-propyl group, an isopropyl group, an n-butyl group, an isobutyl group, a tert-butyl group, a pentyl group, a sec-pentyl group, a tert-pentyl group, a hexyl group, a heptyl group, an octyl group, a 2-ethylhexyl group, a nonyl group and a decyl group.
As the halogen atom, there may be mentioned fluorine, chlorine, bromine and iodine.

In the above formulas (10), (11), (12), (13), (14), (15) and (16) showing the alicyclic group (L), examples of the C₁ to C₁₀ alkyl group represented by Y, Y¹ and Y² include a methyl group, an ethyl group, an n-propyl group, an isopropyl group, an n-butyl group, an isobutyl group, a tert-butyl group, a pentyl group, a sec-pentyl group, a tert-pentyl group, a hexyl group, a heptyl group, an octyl group, a 2-ethylhexyl group, a nonyl group and a decyl group.
Specific examples of the compound forming the alicyclic group (L) are as follows:

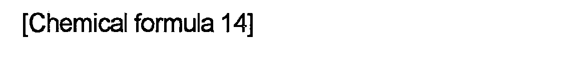

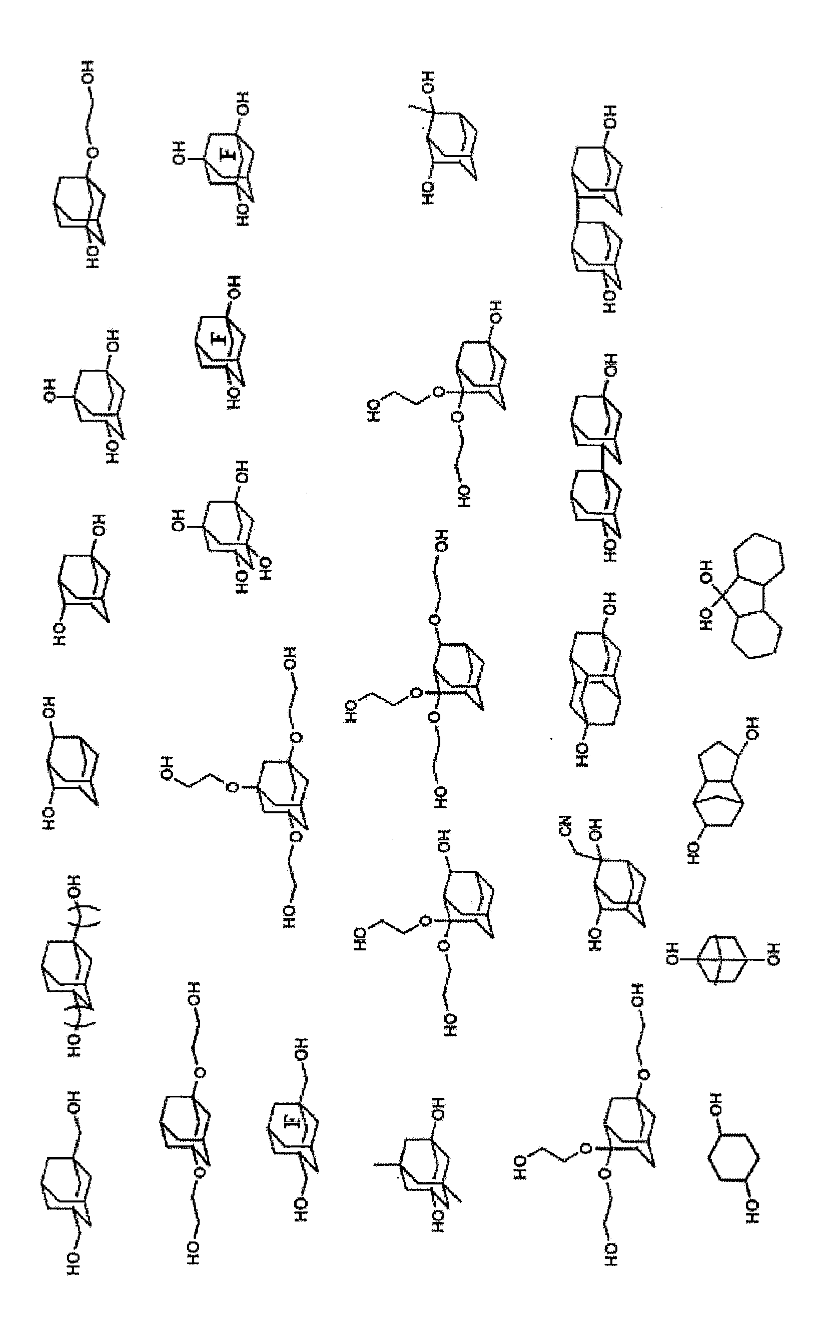

In the above formulas (17) and (18) showing the terminal group (Z), examples of the C₁ to C₃₀ alkyl group represented by R⁷ include a methyl group, an ethyl group, an n-propyl group, an isopropyl group, an n-butyl group, an isobutyl group, a tert-butyl group, a pentyl group, a sec-pentyl group, a tert-pentyl group, a hexyl group, a heptyl group, an octyl group, a 2-ethylhexyl group, a nonyl group, a decyl group, a dodecyl group, a tetradecyl group, a hexadecyl group, an octadecyl group, an icosyl group, a docosyl group, a tetracosyl group, a hexacosyl group, an octacosyl group and a triacontyl group.
Examples of the C₅ to C₃₀ cycloalkyl group represented by R⁷ include a cyclopentyl group, a methylcyclopentyl group, an ethylcyclopentyl group, a cyclohexyl group, a methylcyclohexyl group, an ethylcyclohexyl group, a cyclooctyl group, a methylcyclooctyl group, an ethylcyclooctyl group, a norbornyl group, an adamantyl group, a biadamantyl group and diadamantyl group.
Shown below are examples of the lactone group, ketone group, ether group, ester group and thioester group.
In the formulas, n, m and l are the same as defined above.

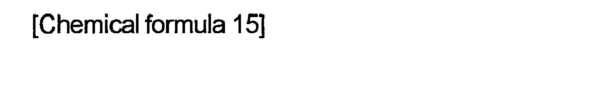

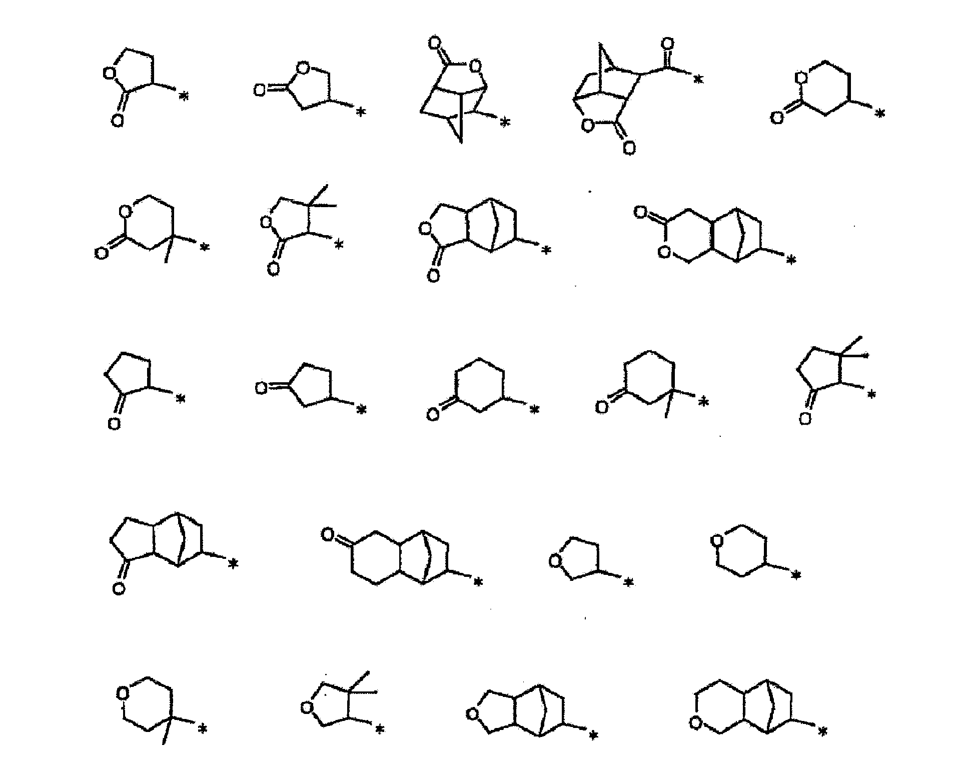

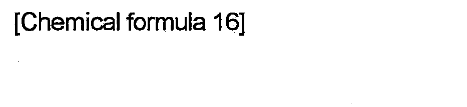

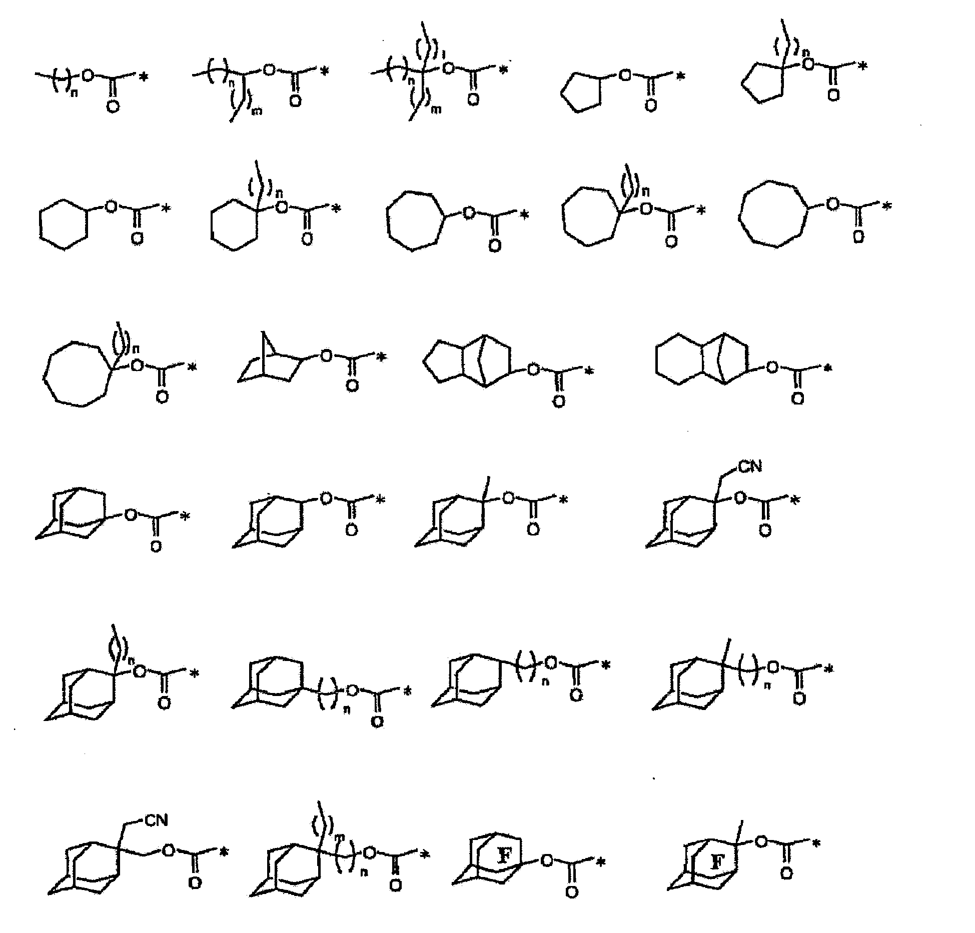

As the halogen atom represented by X⁴ or X⁵ in the compounds of the general formulas (19) and (20) and the compounds of the general formulas (21) and (22), there may be mentioned fluorine, chlorine, bromine and iodine.
As the alkylsulfonyloxy group, there may be mentioned, for example, a methanesulfonyloxy group, an ethanesulfonyloxy group, an n-propanesulfonyloxy group, an isopropanesulfonyloxy group, an n-butanesulfonyloxy group, an isobutanesulfonyloxy group, a pentanesulfonyloxy group and an octanesulfonyloxy group.
As the perfluoroalkylsulfonyloxy group, there may be mentioned, for example, a trifluoromethanesulfonyloxy group, a perfluoroethanesulfonyloxy group, a perfluoropropanesulfonyloxy group, a perfluorobutanesulfonyloxy group, a perfluoropentanesulfonyloxy group and a perfluorooctanesulfonyloxy group.
As the alkyl-substituted phenylsulfonyloxy group, there may be mentioned, for example, a paratoluenesulfonyloxy group, a 4-ethylbenzenesulfonyloxy group, a xylenesulfonyloxy group, a 2-mesitylenesulfonyloxy group, a 4-nitrophenylsulfonyloxy group, a 4-cyanophenylsulfonyloxy group, a 4-trifluoromethylsulfonyloxy group and a 4-chloromethylphenylsulfonyloxy group.

Specific examples of the polymerizable compound of the present invention represented by the general formula (1) and the general formula (19) are shown below. In the formulas, R⁰ represents a hydrogen atom, a fluorine atom, a methyl group, an ethyl group or a trifluoromethyl group, F represents a perfluoroalkylsulfonyloxy group, and n is the same as defined above.

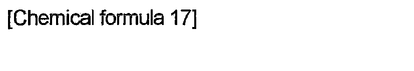

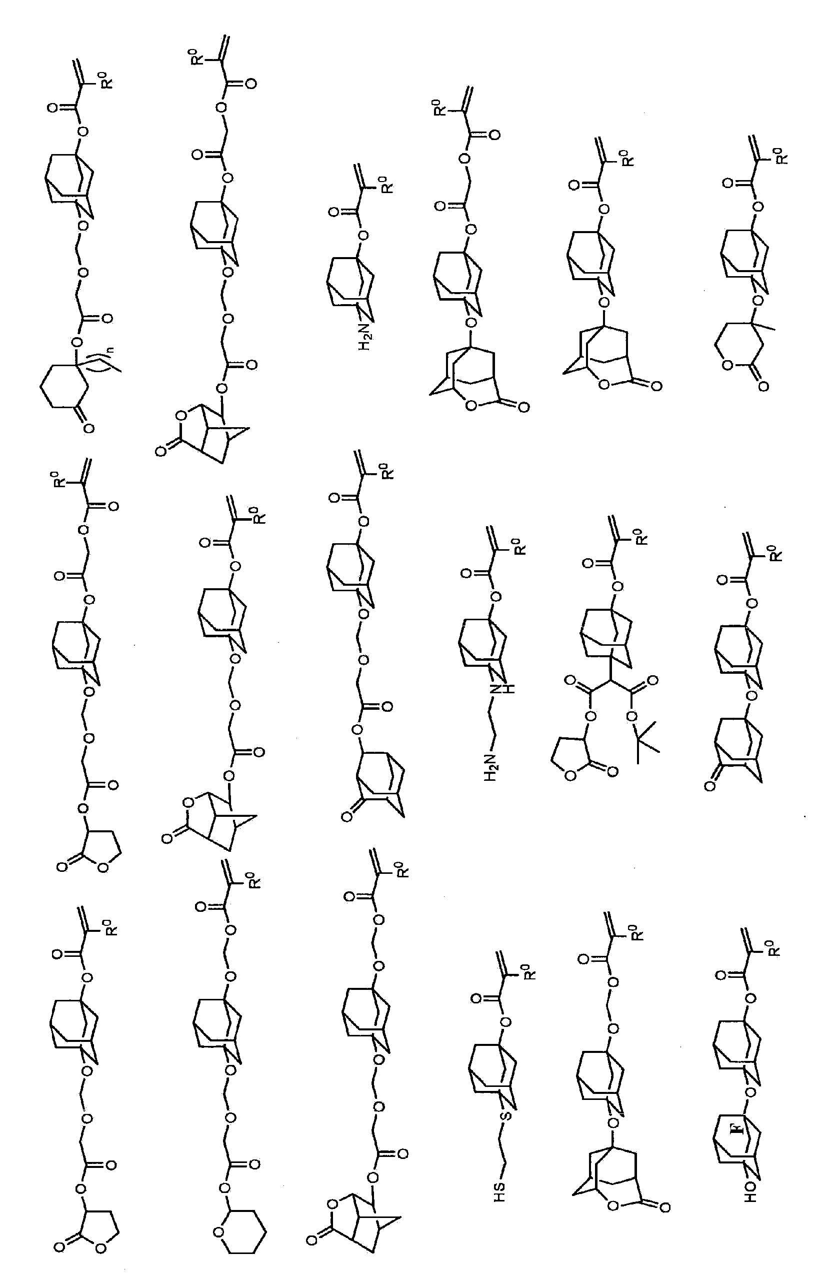

Particularly preferred polymerizable compounds are 3-[(2,6-norbornanecarbolactone-5-yl)oxy]-1-adamantyl methacrylate, 3-[(2-oxotetrahydrofuran- 3-yl)oxy]-1-adamantyl methacrylate, 3-(2-ethoxy-1-methyl-2-oxoethoxy)-1-adamantyl methacrylate, and 3-(2-tert-butyloxy-2-oxoethoxy)-1-adamantyl methacrylate.

As a process for producing the polymerizable compound of the present invention represented by the general formula (19), there may be mentioned a method in which a compound represented by the general formula (20) is reacted with a compound represented by the general formula (21) or a method in which a compound represented by the general formula (22) is reacted with a compound represented by the general formula (23).
In the case of an etherization reaction, for example, the reaction may be carried out at a temperature of -200 to 200 °C, preferably 100 to 150 °C.
When the reaction temperature is excessively low, the reaction rate is so slow that a long reaction time is required. When the reaction temperature is excessively high, production of by- product polymers increases.
The reaction pressure is generally 0.01 to 10 MPa (absolute pressure), preferably ambient pressure to 10 Mpa.
When the reaction pressure is excessively high, a specific apparatus must be used. This is economically unfavorable.
The reaction time is generally 1 to 48 hours.
A base may be used as a reaction promoter.
As the base, there may be mentioned sodium amide, triethylamine, tributylamine, trioctylamine, pyridine, N,N-dimethylaniline, 1,5-diazabicyclo[4.3.0]nonene-5 (DBN), 1,8-diazabicyclo [5,4,0] undecene-7 (DBU), sodium hydroxide, potassium hydroxide, sodium hydride, potassium carbonate, silver oxide, sodium methoxide, potassium t-butoxide, sodium phosphate, sodium monohydrogen phosphate and sodium dihydrogen phosphate.
It is preferred that the reaction be carried out in a suspended state in a solvent, particularly in a dissolved state in a solvent, though the reaction may be performed without a solvent. The solvent is preferably dehydrated.
As the solvent, there may be mentioned, for example, a hydrocarbon solvent such as hexane and heptane, an ether solvent such as diethyl ether, tetrahydrofuran (THF) and dioxane, a halogen-containing solvent such as dichloromethane and carbon tetrachloride, dimethylsulfoxide, N,N-dimethylformamide, N-methylpyrrolidone (NMP) and γ-butyrolactone.
The reaction product may be purified by distillation, crystallization, column chromatography or the like method. Such a purification method is suitably selected in view of the property of the reaction product and the kind of the impurities.

A polymer containing, as a constituting component, the polymerizable compound having an alicyclic structure and a polymerizable group and represented by the general formula (1) or (19) may be a homopolymer or copolymer obtained by polymerizing one or at least two polymerizable compounds each having an alicyclic structure and a polymerizable group and each represented by the general formula (1) or (19). Alternatively, the polymer may be a copolymer obtained by polymerizing at least one polymerizable compound having an alicyclic structure and a polymerizable group and represented by the general formula (1) or (19) and another copolymerizable compound.
The copolymerizable compound to be copolymerized with the polymerizable compound of the general formula (1) or (19) is not specifically limited as long as it has a polymerizable unsaturated double bond and is copolymerized with the polymerizable compound of the general formula (1) or (19). However, when the copolymer obtained is used as a film forming component of the photoresist composition, it is important to select the kind and amount of the copolymerizable compound so that the performance (dry-etching resistance, transparency to light of a short wavelength, etc.) of the photoresist is not adversely affected.

The homopolymer or copolymer obtained from the polymerizable compound of the general formula (1) or (19) generally has a weight average molecular weight of 1,000 to 500,000 in terms of polystyrene equivalent molecular weight as determined by gel permeation chromatography (GPC method).
When the weight average molecular weight is less than 1,000, the film forming property becomes occasionally poor. Too high a weight average molecular weight in excess of 500,000 causes deterioration of developability of a photoresist obtained using such a polymer.
The weight average molecular weight is preferably 2,500 to 50,000 for reasons of well balanced film formability and developability.
The method for producing a homopolymer or copolymer from the polymerizable compound of the general formula (1) or (19) is not specifically limited and can employ radical polymerization, cation polymerization, anion polymerization, coordination polymerization, or the like polymerization method.
Such polymerization methods will be next described.

### Radical polymerization (including thermal polymerization):

The reaction temperature for radical polymerization is generally selected from the range of -100 to 500°C, preferably 0 to 150°C.
When the reaction temperature is excessively low, there is a possibility that the reaction does not smoothly proceed. On the other hand, when the reaction temperature is excessively high, chain transfer occurs so that the molecular weight of the polymer obtained is apt to be low. Additionally, the initiator efficiency is lowered to cause a reduction of the yield.
The monomer concentration is preferably 1 × 10⁻³ mol/L or more. When the monomer concentration is excessively low, it is difficult for the reaction to smoothly proceed.
A reaction solvent may be used, if necessary.
Examples of the reaction solvent include a hydrocarbon solvent such as toluene and a ketone solvent such as methyl isobutyl ketone.
A polymization initiator may be used, if necessary. Examples of the polymerization initiator include an azo compound such as azobisisobutyronitrile, an organic peroxide such as benzoyl peroxide and cumene hydroperoxide, and, further, hydrogen peroxide triethylammonium.
The polymerization initiator is generally used in an amount of 1×10⁻³ to 1×10³ mole %, preferably 1×10⁻¹ to 10 mole %, based on the monomer.
The reaction time depends upon the reaction temperature, kind of the monomer and various other conditions but is generally from several minutes to 48 hours, preferably from 30 minutes to 10 hours.

### Cation polymerization:

The reaction temperature for cation polymerization is generally selected from the range of -100 to 200 °C, preferably 0 to 100°C.
When the reaction temperature is excessively low, there is a possibility that the reaction does not smoothly proceed. On the other hand, when the reaction temperature is excessively high, chain transfer occurs so that there is caused a problem that the molecular weight of the polymer obtained is apt to be low.
The monomer concentration is preferably 1×10⁻³ mol/L or more. When the monomer concentration is excessively low, it is difficult for the reaction to smoothly proceed.
A reaction solvent may be used, if necessary.
Examples of the reaction solvent include a hydrocarbon solvent such as toluene and a halogenated hydrocarbon solvent such as dichloromethane and chloroform.
As a catalyst there may be used, for example, p-toluenesulfonic acid, an ion exchange resin, activated clay, zeolite, clay, aluminum chloride and boron trifluoride.
The catalyst is generally used in an amount of 1×10⁻³ to 1×10³ mole %, preferably 1×10⁻¹ to 10 mole %, based on the monomer.
The reaction time depends upon the reaction temperature, kind of the monomer and various other conditions but is generally from several minutes to 48 hours, preferably from 30 minutes to 10 hours.

### Anion polymerization:

The reaction temperature for anion polymerization is generally selected from the range of -200 to 300°C, preferably -100 to 150°C.
When the reaction temperature is excessively low, there is a possibility that the reaction does not smoothly proceed. On the other hand, when the reaction temperature is excessively high, chain transfer occurs so that the molecular weight of the polymer obtained is apt to be low.
The monomer concentration is preferably 1×10⁻³ mol/L or more. When the monomer concentration is excessively low, it is difficult for the reaction to smoothly proceed.
A reaction solvent may be used, if necessary.
Examples of the reaction solvent include a hydrocarbon solvent such as toluene and an ether solvent such as tetrahydrofuran.
As a polymerization initiator there may be used, for example, alkyllithium, alkylzinc and potassium-kethyl.
The polymerization initiator is generally used in an amount of 1×10⁻³ to 1×10³ mole %, preferably 1×10⁻¹ to 10 mole %, based on the monomer.
The reaction time depends upon the reaction temperature, kind of the monomer and various other conditions but is generally from several minutes to 48 hours, preferably from 30 minutes to 10 hours.

### Coordination polymerization:

The reaction temperature for coordination polymerization is generally selected from the range of -100 to 200 ° C, preferably 0 to 100°C.
When the reaction temperature is excessively low, there is a possibility that the reaction does not smoothly proceed. On the other hand, when the reaction temperature is excessively high, chain transfer occurs so that the molecular weight of the polymer obtained is apt to be low. Also caused is a problem that the catalyst is quickly deactivated.
The monomer concentration is preferably 1 × 10⁻³ mol/L or more. When the monomer concentration is excessively low, it is difficult for the reaction to smoothly proceed.
A reaction solvent may be used, if necessary.
Examples of the reaction solvent include a hydrocarbon solvent such as toluene, cyclohexane and heptane and a halogenated hydrocarbon solvent such as dichloromethane and chloroform.
As a catalyst, there may be used, for example, a Ziegler-Natta catalyst using in combination titanium tetrachloride or zirconium tetrachloride and an alkyl aluminum cocatalyst such as methyl aluminoxane, and a metallocene catalyst using in combination zirconocene halide or titanocene halide and an alkyl aluminum cocatalyst such as methyl aluminoxane.
The catalyst is generally used in an amount of 1×10⁻³ to 1×10³ mole %, preferably 1×10⁻¹ to 10 mole %, based on the monomer.
The reaction time depends upon the reaction temperature, kind of the monomer and various other conditions but is generally from several minutes to 48 hours, preferably from 30 minutes to 10 hours.
The homopolymer or copolymer obtainable from the polymerizable compound of the general formula (1) or (19) of the present invention has an alicyclic structure in its molecule and is excellent in dry-etching resistance and in transparency to a short wavelength light such as far ultraviolet light. Therefore, such a homopolymer or copolymer is suited as a film forming component for a photoresist composition for use with an excimer laser beam, particularly an ArF excimer laser beam or an F₂ excimer laser beam.

The photoresist composition of the present invention, which contains a polymer including as a constituting component the polymerizable compound having an alicyclic structure and a polymerizable group and represented by the general formula (1) or (19), comprises the polymer including as a constituting component the polymerizable compound represented by the general formula (1) or (19) as a film forming component. Additionally, the photoresist composition can contain a compound capable of forming an acid upon irradiation with a radiation ray (hereinafter referred to as "acid generator").

The acid generator is not specifically limited and may be suitably selected, as required, from those customarily used in the field of the conventional chemically amplified type photoresists. Particularly preferred acid generator is a compound which is capable of generating an add when irradiated with a light having a wavelength of 300 nm or less, preferably 200 nm or less, which gives such a mixture with a film forming component that is fully soluble in an organic solvent to give a solution, and which allows the solution to form a uniform coated film by spin coating or the like film forming method.
As the acid generator, there may be mentioned, for example, onium salts, diazomethane derivatives, glyoxime derivatives, β-ketosulfone derivative, disulfone derivatives, nitrobenzyl sulfonate derivatives, sulfonic acid ester derivatives and imide-yl sulfonate derivatives.

As the onium salt, there may be mentioned, for example, sulfonium salts typical example of which are triphenylsulfonium salt derivatives, iodonium salts, phosphonium salts, diazonium salts and ammonium salts. Specific examples of the onium salt include triphenylsulfonium trifluoromethanesulfonate, triphenylsulfonium hexafluorophosphate, cyclohexylmethyl(2-oxocyclohexyl)sulfonium trifluoromethanesulfonate, dicyclohexyl(2-oxocyclohexyl)sulfonium trifluoromethanesulfonate, dimethyl(2-oxocyclohexyl)sulfonium trifluoromethanesulfonate, (4-methoxyphenyl)diphenylsulfonium trifluoromethanesulfonate, diphenyliodonium trifluoromethanesulfonate, diphenyliodonium hexafluorophosphate and (4-methoxyphenyl)phenyliodonium trifluoromethanesulfonate.
Examples of the diazomethane derivative include bis(benzenesulfonyl)diazomethane, bis(p-toluenesulfonyl)diazomethane, bis(cyclohexylsulfonyl)diazomethane and bis(n-butylsulfonyl)diazomethane. Examples of the glyoxime derivative include bis-O-(p-toluenesulfonyl)-α-dimethylglyoxime, bis-O-(p-toluenesulfonyl)-α-diphenylglyoxime and bis-O-(n-butanesulfonyl)-α-dimethylglyoxime. Examples of the β-ketosuifone derivative include 2-cyclohexylcarbonyl-2-(p-toluenesulfonyl)propane and 2-isopropylcarbonyl-2-(p-toluenesulfonyl)propane. Examples of the disulfone derivative include diphenyl disulfone and dicyclohexyl disulfone.

Examples of the nitrobenzylsulfonate derivative include 2,6-dinitrobenzyl p-toluenesulfonate and 2,4-dinitrobenzyl p-toluenesulfonate. Examples of the sulfonic acid ester derivative include 1,2,3-tris(methanesulfonyloxy)benzene and 1,2,3 tris(trifluoromethanesulfonyloxy)benzene. Examples of the imide-yl sulfonate derivatives include phthalimide-yl triflate, phthalimide-yl tosylate and 5-norbomene-2,3-dicarboximide-yl triflate.
Among the above acid generators, onium salts are suitably used.
In the present invention, the acid generator may be used singly or two or more kinds of the acid generators may be used in combination.
The compounding amount of the acid generator is not specifically limited but is generally selected from the range of 0.2 to 25 % by mass based on a total amount of the film forming component, the acid generator and hereinafter described dissolution inhibitor which is optionally incorporated.
When the amount of the acid generator is less than 0.2 % by mass, the sensitivity considerably decreases to cause a difficulty to form a pattern. When the amount exceeds 25 % by mass, it is difficult to form a uniform film and, moreover, residues (scum) are apt to form after development.
The photoresist composition of the present invention may contain the dissolution inhibitor and an acid diffusion controlling agent, if necessary

The dissolution inhibitor is not specifically limited and can be suitably selected from various compounds as required. Particularly preferred inhibitor is a compound which has a group decomposed by an acid, which little absorbs a light having a wavelength of 300 nm or less, particularly 200 nm or less, which gives such a mixture with a film forming component that is fully soluble in an organic solvent to give a solution, and which allows the solution to form a uniform coated film by spin coating or the like film forming method.
As the group decomposable by an acid, there may be mentioned, for example, an alkyl group such as a tert-butyl group, an ethyl group and a propyl group, an aralkyl group such as a benzyl group and a phenethyl group, a tetrahydrofuran-2-yl group, a tetrahydropyran-2-yl group, a 4-methoxytetrahydropyran-4-yl group, a 1-butoxyethyl group, a 1-ethoxyethyl group, a 1-propoxyethyl group and a 3-oxocyclohexyl group.
Particularly preferred dissolution inhibitor is an alicyclic compound such as norbornane or adamantane having a carboxyl group or hydroxyl group whose hydrogen atom is substituted with the above-described group decomposable by an acid.
These compounds are converted into compounds having the corresponding free carboxyl group or hydroxyl group after the decomposition with an acid.

Examples of the dissolution inhibitor include compounds obtainable by substituting the hydrogen atom of the carboxyl group of 2-norbornanecarboxylic acid, 2-norbomaneacetic acid, 2,3-norbornanedicarboxylic acid, 3-noradamantanecarboxylic acid and the like acid with the above-described group capable of being decomposed with an acid, and compounds obtainable by substituting the hydrogen atom of the hydroxyl group of 2,3-norbomanediol, 2-norbomanemethanol, 2,2-norbomanedimethanol, norborneol and the like with the above-described group capable of being decomposed with an acid.
In the present invention, the dissolution inhibitor may be used singly or two or more kinds of the dissolution inhibitors may be used in combination.
The compounding amount of the dissolution inhibitor is generally selected from the range of 2 to 60 parts by mass per 100 parts by mass of the film forming component.
When the amount of the dissolution inhibitor is less than 2 parts by mass, the dissolution inhibiting effect is not sufficiently obtained so that there is a possibility that the formation of a pattern becomes difficult. An amount of the dissolution inhibitor in excess of 60 parts by mass is not preferable, because the proportion of the film forming component is so low that the heat resistance and etching resistance of the resist pattern are lowered.
From the standpoint of the pattern formability and the heat resistance and etching resistance of the resist pattern, the compounding amount of the dissolution inhibitor is preferably in the range of 10 to 50 parts by mass.

As the acid diffusion preventing agent, various amines may be used. Specific examples of the acid diffusion preventing agent include aliphatic amines such as trimethylamine, ethylamine, diethylamine, triethylamine, n-propylamine, di-n-propylamine, tri-n-propylamine, aromatic amines such as benzylamine, aniline, N-methylaniline and N,N-dimethylaniline, and heterocyclicamines such as pyridine, 2-methylpyridine, 2-ethylpyridine and 2,3-dimethylpyridine.
The acid diffusion preventing agent may be used singly or two or more kinds of the acid generators may be used in combination.
The compounding amount of the acid diffusion preventing agent is generally in the range of 0.01 to 1 % by mass, preferably 0.05 to 0.5 % by mass, based on the amount of the film forming component from the standpoint of formability and sensitivity of the resist pattern.

In use, the photoresist composition of the present invention is preferably used in the form of a solution in which the above-described components are dissolved in a suitable solvent.
Examples of the solvent include halogenated hydrocarbons such as monochlorobenzene and ethylene dichloride; ketones such as acetone, methyl ethyl ketone, cyclohexanone and methyl isoamyl ketone; polyhydric alcohols and their derivatives such as ethylene glycol, ethylene glycol monoacetate, diethylene glycol, diethylene glycol monoacetate, propylene glycol, propylene glycol monoacetate, dipropylene glycol, dipropylene glycol monoacetate, and monomethyl ether, monoethyl ether, monopropyl ether, monobutyl ether and monophenyl ether thereof; cyclic ethers such as dioxane; and esters such as methyl lactate, ethyl lactate, methyl acetate, ethyl acetate, butyl acetate, methyl pyruvate, ethyl pyruvate, methyl methoxypropionate and ethyl ethoxypropionate.
The above solvents may be used singly or as a mixture of two or more thereof.
If desired, the photoresist composition of the present invention may further contain miscible additives, such as an additional resin for improving the property of the resist film, a plasticizer, a stabilizer, a colorant and a surfactant, which are customarily used additives.

### Examples

The present invention will be next described in more detail by way of examples but is not limited thereto in any way.

### Example 1

### Synthesis of 3-[(2,6-norbornanecarbolactone-5-yl)oxy]-1-adamantyl methacrylate:

In a 50 mL three-necked, eggplant-shaped flask equipped with a stirrer, a condenser and an air inlet tube, 3.15 g (10 mmol) of 3-methanesulfonyloxy-1-adamantyl methacrylate, 12.33 g (80 mmol) of 5-hydroxy-2,6-norbornanecarbolactone, 2.22 g (12 mmol) of tri-n-butylamine and 3 mg (1000 ppm by mass) of methoquinone were charged and heated to 120°C with stirring.
The mixture was then stirred for 2 hours while maintaining the temperature at 120°C. It was confirmed by gas chromatography (GC) that 3-methanesulfonyloxy-1-adamantyl methacrylate completely disappeared.
The resulting reaction solution was then cooled to room temperature, to which 40 mL of distilled water and 30 mL of diethyl ether were added. Using a separatory funnel, the desired product was extracted into an organic layer.
Then, 30 ml of distilled water was added to the organic layer and the organic layer was washed twice therewith. Succeedingly, 20 mL of 1 mole/L hydrochloric acid were added to the organic layer and the organic layer was washed therewith.
Then, 20 ml of saturated aqueous saline solution was added to the organic layer and the organic layer was washed twice therewith. Thereafter the aqueous layer was dried for 2 hours over anhydrous magnesium sulfate.
The organic layer was filtered to remove magnesium sulfate and the solvent was removed by distillation under vacuo to obtain the crude title product (yield: 3.25 g, purity: 91.7%).
The crude product was homogeneously dissolved in 3 mL of methanol, to which 0.1 mL of distilled water was added. Crystals were precipitated when the solution was cooled to 0°C.
The crystals were collected by filtration and washed with cold distilled water. The solvent was removed under vacuo to obtain the title product in the form of white solid (yield: 2.36 g, purity: 99.8 %).

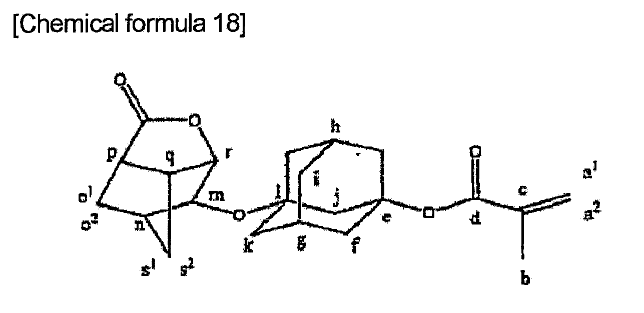

The physical data of thus obtained product are as follows.

### Nuclear magnetic resonance spectroscopy (NMR), solvent: CDCl₃:

¹H-NMR (500 MHz): 1.24-1.27 (2H), 1.30 (1 H, S¹ or S²), 1.36-1.54 (8H), 1.55 (1 H, S¹ or S²), 1.57(1 H), 1.63 (1 H, o¹ or o²), 1.66 (1 H), 1.73 (1 H), 1.76 (1 H, n), 1.88 (1 H, o¹ or o²), 1.93 (3H, b), 2.04 (1H), 2.26 (1 H, p), 2.66 (1 H, q), 3.37 (1 H, m), 4.22 (1 H, r), 5.58 (1 H, a²), 6.15 (1H, a¹)
¹³C-NMR (127 MHz): 18.0 (b), 21.3 (g or h), 23.3 (g or h), 27.2 (o), 30.9 (q), 33.5 (s), 38.1 (i), 41.6 (n), 42.4 (f or k), 43.4 (f or k), 46.3 (j), 46.8 (p), 62.5 (l), 74.6 (m), 75.4 (e), 85.5 (r), 125.2 (a), 136.1 (c), 167.2 (d), 178.9 (t)

### Gas chromatography - Mass spectrometric analysis (GC-MS), EI:

372 (M⁺, 3.5 %), 286 (12.8 %), 219 (48.3 %), 133 (18.2%), 81 (16.8 %), 69 (100 %)

### Example 2

### Synthesis of 3-[(2-oxotetrahydrofuran-3-yl)oxy]-1-adamantyl methacrylate:

In a 50 mL three-necked, eggplant-shaped flask equipped with a stirrer, a condenser and an air inlet tube, 3.15 g (10 mmol) of 3-methanesulfonyloxy-1-adamantyl methacrylate, 20.41 g (200 mmol) of α-hydroxy-y-butyrolactone, 1.42 g (10 mmol) of sodium monohydrogen phosphate and 3 mg (1000 ppm by mass) of methoquinone were charged and heated to 60 ° C with stirring.
The mixture was then stirred for 4 hours while maintaining the temperature at 60°C. It was confirmed by gas chromatography (GC) that 3-methanesulfonyloxy-1-adamantyl methacrylate completely disappeared.
The resulting reaction solution was then cooled to room temperature, to which 20 mL of distilled water and 30 mL of diethyl ether were added. Using a separatory funnel, the desired product was extracted into an organic layer.
Then 30 ml of distilled water was added to the organic layer and the organic layer was washed twice therewith. Succeedingly, 20 ml of saturated aqueous saline solution was added to the organic layer and the organic layer was washed twice therewith.
Thereafter the organic layer was dried for 2 hours over anhydrous magnesium sulfate.
The organic layer was filtered to remove magnesium sulfate and the solvent was removed by distillation under vacuo to obtain the crude title product (yield: 2.17 g, purity: 78.1 %).
The crude product was homogeneously dissolved in 2 mL of diethyl ether, to which 5 mL of n-hexane was added. Crystals were precipitated when the solution was cooled to 0°C.
The crystals were collected by filtration and washed with cold n-hexane. The solvent was removed under vacuo to obtain the title product in the form of white solid (yield: 1.45 g, purity: 98.2 %).

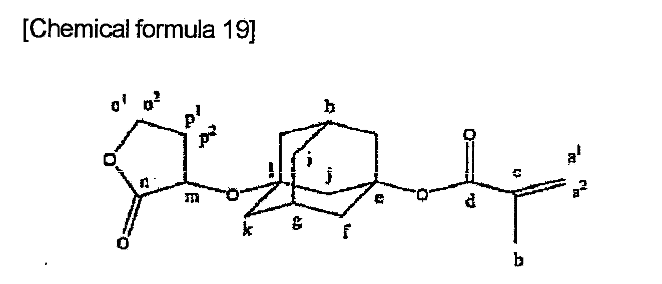

The physical data of thus obtained product are as follows.

### Nuclear magnetic resonance spectroscopy (NMR), solvent CDCl₃:

¹H-NMR (500 MHz): 1.24-1.73 (13H), 1.93 (3H, b), 2.04 (1 H), 2.11 (1 H, p¹ or p²), 2.36 (1 H, p¹ or p²), 3.65 (1 H, m), 4.25 (1 H, o¹ or o²), 4.35 (1 H, o¹ or o²), 5.58 (1 H, a²), 6.15 (1 H, a¹) ¹³C-NMR (127 MHz): 18.0 (b), 21.3 (g or h), 23.3 (g or h), 23.9 (p), 38.1 (i), 42.4 (f or k), 43.4 (f or k), 46.0 (j), 61.6 (l), 65.0 (o), 75.4 (e), 85.8 (m), 125.2 (a), 136.1 (c), 167.2 (d), 173.7 (n)

### Gas chromatography-Mass spectrometric analysis (GC-MS), EI:

320 (M+, 1.0 %), 234 (42.3 %), 219 (25.8 %), 133 (21.9 %), 91 (7.3 %), 69 (100 %)

### Example 3

### Synthesis of 3-(2-ethoxy-1-methyl-2-oxoethoxy)-1-adamantyl methacrylate:

In a 500 mL flask, 30 g (0.095 mol) of 3-methanesulfonyloxy-1-adamantyl methacrylate, 168.34 g (1.425 mol) of ethyl lactate, 19.24 g (0.190 mol) of triethylamine and 500 ppm by mass of a polymerization inhibitor were charged and reacted at 120°C for 10 hours.
The resulting reaction solution was then cooled to room temperature, to which water and hexane were added. Extraction with hexane was then carried out. The hexane extract was washed three times with 200 mL water.
Activated carbon was then added to the washed extract in an amount of 10 % by mass based on the 3-methanesulfonyloxy-1- adamantyl methacrylate used. This was stirred for 1 hour. Then, the activated carbon was removed and, further, the solvent was removed to obtain the title product (yield: 20.27 g, purity: 98.0 %).

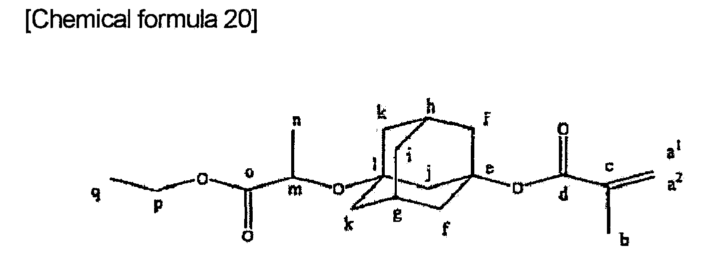

The physical data of thus obtained product are as follows.

### Nuclear magnetic resonance spectroscopy (NMR), solvent: CDCl₃:

¹H-NMR (500 MHz): 1.24-1.27 (2H), 1.30 (3H, q), 1.36 (1 H), 1.39 (3H, n), 1.42-1.73 (10H), 1.93 (3H, b), 2.04 (1 H), 3.85 (1 H, m), 4.12 (2H, p), 5.58 (1 H, a²), 6.15 (1 H, a¹)
¹³C-NMR (127 MHz): 18:0 (b), 14.1 (q), 17.1 (n), 21.3 (g or h), 23.3 (g or h), 38.1 (i), 42.4 (f or k), 43.1 (f or k), 46.0 (j), 62.3 (l), 61.6 (p), 70.9 (m), 75.4 (e), 125.2 (a), 136.1 (c), 167.2 (d), 170.9 (o)

### Gas chromatography - Mass spectrometric analysis (GC-MS), El:

292 (M⁺-C₂H₄, 0.41 %), 263 (9.8 %), 219 (82.1 %), 133 (22.3 %), 91 (6.3 %), 69 (100 %)

### Example 4

### Synthesis of 3-(2-tert-butyloxy-2-oxoethoxy)-1-adamantyl methacrylate:

In a 100 mL flask, 3.95 g (0.0125 mol) of 3-methanesulfonyloxy-1-adamantyl methacrylate, 25 g (0.1875 mol) of t-butyl glycolate, 2.55 g (0.025 mol) of triethylamine and 500 ppm by mass of a polymerization inhibitor were charged and reacted at 120°C for 8 hours.
The resulting reaction solution was then cooled to room temperature, to which water and hexane were added. Extraction with hexane was then carried out. The hexane extract was washed three times with 50 mL water.
The resulting hexane extract was subjected to column chromatography (elution solvent: n-hexane/diethyl ether = 8/1, amount of silica gel: 39.5 g) to obtain the title product (yield: 1.67 g, purity: 99.0 %).

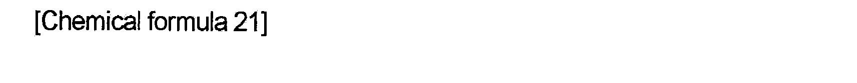

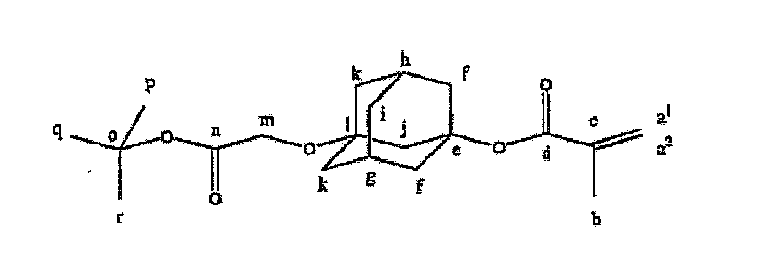

The physical data of thus obtained product are as follows.

### Nuclear magnetic resonance spectroscopy (NMR), solvent: CDCl₃:

¹H-NMR (500 MHz): 1.24-1.36 (3H), 1.40 (9H, p & q & r), 1.42-1.73 (10H), 1.93 (3H, b), 2.04 (1 H), 4.33 (2H, m), 5.58 (1 H, a²), 6.15 (1 H, a¹)
¹³C-NMR (127 MHz): 18.0 (b), 21.3 (g or h), 23.3 (g or h), 28.8 (p & q & r), 38.1 (i), 42.4 (f or k), 42.8 (f or k), 45.7 (j), 62.8 (I), 64.0 (m), 75.4 (e), 82.1 (o), 125.2 (a), 136.1 (c), 167.2 (d), 169.3 (n)

### Gas chromatography - Mass spectrometric analysis (GC-MS), EI:

306 (M⁺-C₃H₈, 0.24 %), 294 (0.15 %), 277 (1.2 %), 219 (73.4 %), 134 (55.4 %), 69 (100 %)

### Example 5

In 30 mL of tetrahydrofuran (THF) were dissolved 9.31 g (25 mmol) of 3-[(2,6-norbornanecarbolactone-5-yl)oxy]-1-adamantyl methacrylate obtained in Example 1 and 5.86 g (25 mmol) of Adamantate MM (manufactured by Idemitsu Kosan Co., Ltd.) as monomers, to which 0.82 g (5 mmol) of azobisbutyronitrile (AIBN) was added as a polymerization initiator.
The mixture was subjected three times to freezing deaeration in the atmosphere of nitrogen at a temperature of liquid nitrogen, followed by reaction in the atmosphere of nitrogen at 60°C for 12 hours.
The reaction was terminated by addition of 2 mL of methanol to the reaction liquid. The resulting reaction mixture was poured dropwise into 300 mL of methanol with stirring for reprecipitation.
The precipitate was filtered on a glass filter and dried under vacuum at 60°C for 3 days to obtain 12.93 g of a polymer.
The polymer was measured for its molecular weight by gel permeation chromatography (GPC method) to reveal that the polystyrene equivalent molecular weight was 12,500 and Mw/Mn was 2.4.

### Example 6

### Synthesis of 2-{[3-(methacryloyloxy)-1-adamantyl]oxy}propanoic acid:

In a 300 mL baffle-plated separable flask equipped with a dropping funnel, a stirring blade and a thermometer, 80 g of methanol, 40 g of water and 16.4 g (0.12 mol) of potassium carbonate were charged and heated with stirring so that the liquid in the flask had a temperature of 50° C. Twenty g (0.06 mol) of 3-(2-Ethoxy-1-methyl-2-oxoethoxy)-1-adamantyl methacrylate obtained in Example 3 was placed in the dropping funnel and was added dropwise. The mixture was reacted for 4 hours after the dropwise addition. Then, 200 mL of water was added to the reaction mixture. One mol/L of Hydrochloric acid was added to the aqueous layer to adjust the pH to 4. Thereafter, the product was extracted with ether. The ether layer was dried over magnesium sulfate, followed by filtration and concentration. The title product was obtained after the concentration as a white solid (yield: 83 %).

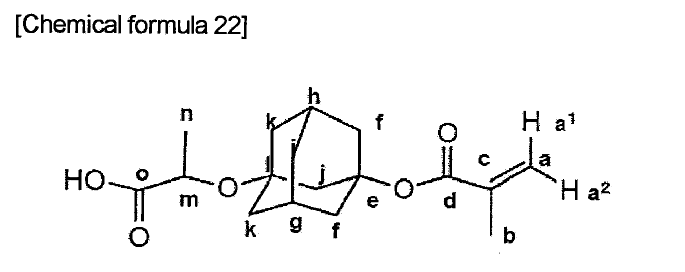

The physical data of thus obtained product are as follows.

### Nuclear magnetic resonance spectroscopy (NMR) solvent: CDCl₃:

¹H-NMR (500 MHz): 1.24-1.30 (2H), 1.36 (1 H), 1.39 (3H, n), 1.42-1.73 (10H), 1.93 (3H, b), 2.04 (1H), 3.85 (1H, m), 5.58 (1H, a2), 6.15 (1H, a1)
¹³C-NMR (127 MHz): 17.1 (q), 17.1 (n), 18.0 (b), 21.3 (g or h), 23.3 (g or h), 38.1 (i), 42.4 (f or k), 43.1 (f or k), 46.0 (j), 62.3 (1), 61.6 (p), 70.9 (m), 75.4 (e), 125 (a), 136.1 (c), 167.2 (d), 170.9(o)

### Example 7

### Synthesis of 3-{2-[(t-butyloxy)oxy]-1-methyl-2-oxoethoxy}-1-adamantyl methacrylate:

In a 1 L three-necked flask equipped with a dropping funnel, a stirring blade and a thermometer, 5.26 g (0.071 mol) of t-butanol, 0.79 g (0.0065 mol) of dimethylaminopyridine, 20 g (0.065 mol) of 2-{[3-(methacryloyloxy)-1-adamantyl]oxy}propanoic acid obtained in Example 6, and 100 mL of hexane were charged. To the resulting mixture, 14.65 g (0.071 mol) of dicyclohexylcarbodiimide (DCC) dissolved in 170 mL of hexane was added dropwise. The reaction was continued for 8 hours. After the termination of the reaction, water was added to the reaction solution to extract the organic layer. The extracted organic layer was washed five times with 100 mL of water. The washed organic layer was concentrated to obtain the title product as a transparent liquid (yield: 76 %).

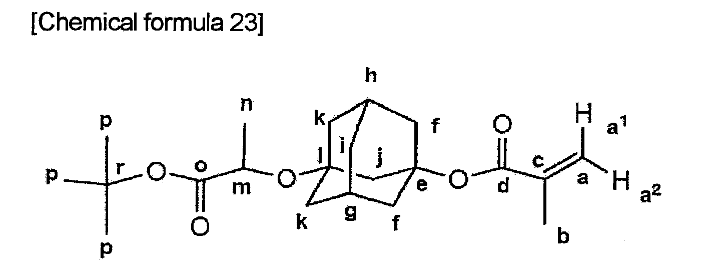

The physical data of thus obtained product are as follows.

### Nuclear magnetic resonance spectroscopy (NMR), solvent: CDCl₃:

¹H-NMR (500 MHz): 1.24-1.36 (3H), 1.39 (3H, n), 1.40 (9H, p), 1.42-1.73 (10H), 1.93 (3H, b), 2.04 (1H), 3.85 (1H, m), 5.58 (1H, a2), 6.15 (1H, a1)
¹³C-NMR (127 MHz): 17.1 (q), 17.1 (n), 18.0 (b), 21.3 (g or h), 23.3 (g or h), 28.8 (p), 38.1 (i), 42.4 (f or k), 43.1 (f or k), 46.0 (j), 62.3 (I), 61.6 (p), 70.9 (m), 75.4 (e), 82.4 (r), 125 (a), 136.1 (c), 167.2 (d), 170.9 (o)

### Example 8

### Synthesis of 3-{2-[(2-methyl-2-cyclohexyl)oxy]-1-methyl-2-oxoethoxy}-1-adamantyl methacrylate:

The procedures of Example 7 were carried out in the same manner using the same apparatus, the same amounts of reagents and the same conditions with the exception that 8.11 g (0.071 mol) of methylcyclohexanol was used in place of t-butanol, thereby obtaining the title product as a transparent liquid with a yield of 74 %.

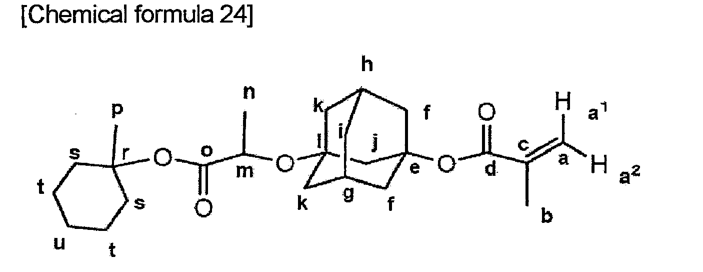

### Nuclear magnetic resonance spectroscopy (NMR), solvent: CDCl₃:

¹H-NMR (500 MHz): 1.24-1.30 (2H), 1.36 (1H), 1.39 (3H, n), 1.42-1.76 (23H), 1.93 (3H, b), 2.04 (1H), 3.85 (1H, m), 5.58 (1H, a2), 6.15 (1H, a1)
¹³C-NMR (127 MHz): 17.1 (q), 17.1 (n), 18.0 (b), 19.5 (t), 21.3 (g or h), 23.3 (g or h), 24.1 (p), 28.3 (u), 38.1 (i), 40.6 (s), 42.4 (f or k), 43.1 (f or k), 46.0 (j), 62.3 (1), 61.6 (p), 70.9 (m), 75.4 (e), 88.7 (r),125 (a), 136.1 (c), 167.2 (d), 170.9 (o)

### Example 9

### Synthesis of 3-{2-[(2-ethyl-2-cyclohexyl)oxy]-1-methyl-2-oxoethoxy}-1-adamantyl methacrylate:

The procedures of Example 7 were carried out in the same manner using the same apparatus, the same amounts of reagents and the same conditions with the exception that 9.10 g (0.071 mol) of ethylcyclohexanol was used in place of t-butanol, thereby obtaining the title product as a transparent liquid with a yield of 76 %.

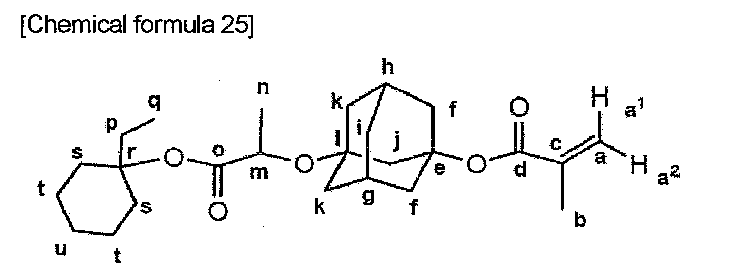

### Nuclear magnetic resonance spectroscopy (NMR), solvent: CDCl₃:

¹H-NMR (500 MHz): 0.96 (3H, q), 1.24-1.30 (2H), 1.36 (1H),1.39 (3H, n), 1.42-1.76 (23H), 1.93 (3H, b), 2.04 (1 H), 3.85 (1 H, m), 5.58 (1 H, a2), 6.15 (1 H, a1)
¹³C-NMR (127 MHz): 5.9 (q), 17.1 (q), 17.1 (n), 18.0 (b), 19.5 (t), 21.3 (g or h), 23.3 (g or h), 24.1 (p), 28.3 (u), 38.1 (i), 40.6 (s), 42.4 (f or k), 43.1 (f or k), 46.0 (j), 62.3 (l), 61.6 (p), 70.9 (m), 75.4 (e), 88.7 (r), 125 (a), 136.1 (c), 167.2 (d), 170.9 (o)

### Example 10

### Synthesis of 3{2-[(cyclohexyloxy)methoxy]-1-methyl-2-oxoethoxy}-1-adamantyl methacrylate:

In a 1 L three-necked flask equipped with a dropping funnel, a stirring blade and a thermometer, 7.24 g (0.072 mol) of triethylamine, 20 g (0.065 mol) of 2-{[3-(methacryloyloxy)-1-adamantyl]oxy}propanoic acid obtained in Example 6, and 100 mL of ethyl acetate were charged. To the resulting mixture, 10.7 g (0.072 mol) of 2-(chloromethoxy)cyclohexane dissolved in 10 mL of ethyl acetate was added dropwise in an ice bath. The reaction was continued for 2 hours. After the termination of the reaction, water was added to the reaction solution to extract the organic layer. The organic layer was washed three times with 100 mL of water. The washed organic layer was concentrated to obtain the title product as a transparent liquid (yield: 82 %).

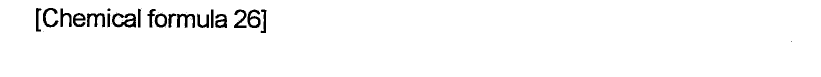

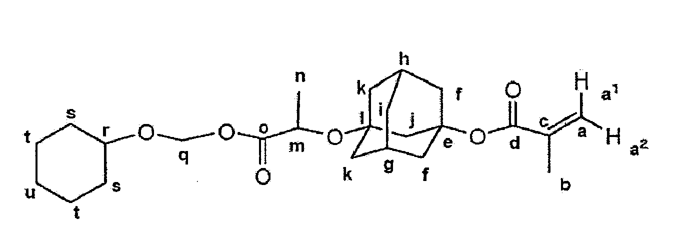

### Nuclear magnetic resonance spectroscopy (NMR), solvent: CDCl₃:

¹H-NMR (500 MHz): 1.24-1.30 (2H), 1.36 (1 H), 1.38 (3H, n), 1.39-1.73 (20H), 1.93 (3H, b), 2.04 (1 H), 2.79 (1 H, r), 3.85 (1 H, m), 5.58 (1 H, a2), 6.15 (1 H, a1), 6.16 (2H, q)
¹³C-NMR (127 MHz): 17.1 (q), 17.1 (n), 18.0 (b), 21.3 (g or h), 22.1 (t), 23.3 (g or h), 28.3 (u), 34.0 (s), 38.1 (i), 42.4 (f or k), 43.1 (f or k), 46.0 (j), 62.3 (l), 61.6 (p), 70.9 (m), 75.4 (e), 78.5 (r), 91.2 (q), 125 (a), 136.1 (c), 167.2 (d), 170.9 (o)

### Example 11

### Synthesis of 3-{2-[(2-ethyl-2-cyclopentyl)oxy]-1-methyl-2-oxoethoxy}-1-adamantyl methacrylate:

The procedures of Example 7 were carried out in the same manner using the same apparatus and the same amounts of reagents with the exception that 8.11 g (0.071 mol) of ethylcyclopentanol was used in place of t-butanol, thereby obtaining the title product as a transparent liquid with a yield of 90 %.

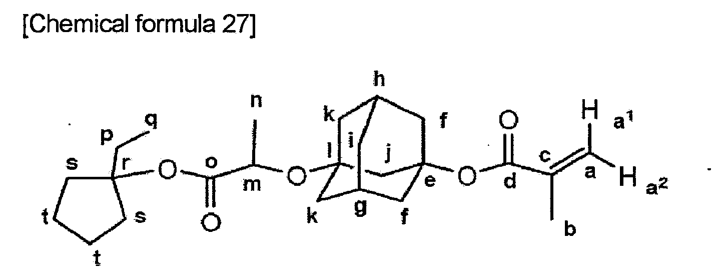

### Nuclear magnetic resonance spectroscopy (NMR), solvent: CDCl₃:

¹H-NMR (500 MHz): 0.96 (3H, q), 1.24-1.30 (2H), 1.36 (1 H), 1.39 (3H, n), 1.42-1.84 (18H), 1.93 (3H, b), 2.04 (1 H), 3.85 (1 H, m), 5.58 (1 H, a2), 6.15 (1 H, a1)
¹³C-NMR (127 MHz): 5.9 (q), 17.1 (q), 17.1 (n), 18.0 (b), 21.3 (g or h), 22.2 (t), 23.3 (g or h), 28.3 (u), 31.6 (p), 38.1 (i), 39.6 (s), 42.4 (f or k), 43.1 (f or k), 46.0 (j), 62.3 (l), 61.6 (p), 70.9 (m), 75.4 (e), 88.7 (r), 125 (a), 136.1 (c), 167.2 (d), 170.9 (o)

### Example 12

### Synthesis of 3-{2-[(2-methyl-2-adamantyl)oxy]-1-methyl-2-oxoethoxy}-1-adamantyl methacrylate:

The procedures of Example 7 were carried out in the same manner using the same apparatus, the same amounts of reagents and the same conditions with the exception that 11.80 g (0.071 mol) of 2-methyladamantane-2-ol was used in place of t-butanol, thereby obtaining the title product as a transparent viscous liquid with a yield of 65 %.

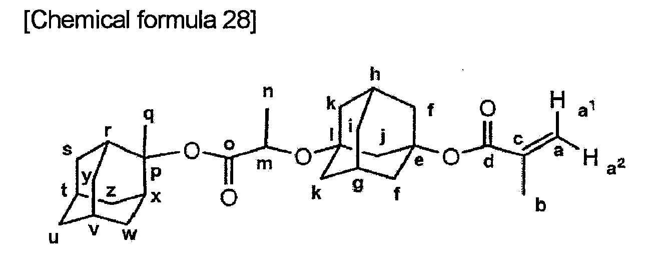

### Nuclear magnetic resonance spectroscopy (NMR), solvent: CDCl₃:

¹H-NMR (500 MHz): 1.18 (5H), 1.36 (1H), 1.38 (3H, n), 1.39-1.91 (23H), 1.93 (3H, b), 2.04 (1 H), 3.85 (1 H, m), 5.58 (1 H, a2), 6.15 (1 H, a1)
¹³C-NMR (127 MHz): 17.1 (n), 18.0 (b), 19.4 (q), 21.3 (g or h) 22.2 (t), 23.3 (g or h), 28.3 (u), 29.3 (s, y, v or w), 31.5 (t or v), 31.6 (p), 37.8 (u), 38.1 (i), 39.6 (s), 40.0 (r or x), 42.4 (f or k), 43.1 (f or k), 46.0 (j), 62.3 (l), 70.9 (m), 75.4 (e), 83.4 (p), 88.7 (r), 125 (a), 136.1 (c), 167.2 (d), 170.9(o)

### Example 13

### Synthesis of 3-{2-[(2-ethyl-2-adamantyl)oxy]-1-methyl-2-oxoethoxy}-1-adamantyl methacrylate:

The procedures of Example 7 were carried out in the same manner using the same apparatus, the same amounts of reagents and the same conditions with the exception that 12.80 g (0.071 mol) of 2-ethyladamantane-2-ol was used in place of t-butanol, thereby obtaining the title product as a transparent viscous liquid with a yield of 60 %.

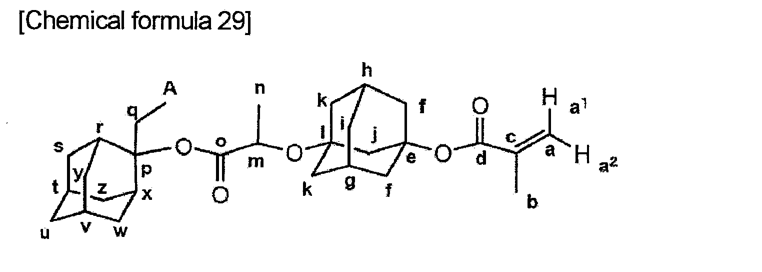

### Nuclear magnetic resonance spectroscopy (NMR), solvent: CDCl₃:

¹H-NMR (500 MHz): 0.96 (3H, A), 1.18 (5H), 1.36 (1 H), 1.38 (3H, n),1.39-1.91 (23H), 1.93 (3H, b), 2.04 (1 H), 3.85 (1 H, m), 5.58 (1 H, a2), 6.15 (1 H, a1)
¹³C-NMR (127 MHz): 6.5 (A), 17.1 (n), 18.0 (b), 21.3 (g or h), 22.2 (t), 23.3 (g or h), 27.2 (q), 28.3 (u), 29.3 (s, y, v or w), 31.5 (t or v), 31.6 (p), 37.8 (u), 38.1 (i), 39.6 (s), 40.0 (r or x), 42.4 (f or k), 43.1 (f or k), 46.0 (j), 62.3 (l), 70.9 (m), 75.4 (e), 83.4 (p), 88.7 (r), 125 (a), 136.1 (c), 167.2 (d), 170.9 (o)

### Example 14

### Synthesis of 3-{2-[(cyclohexyl)oxy]-1-methyl-2-oxoethoxy}-1-adamantyl methacrylate:

The procedures of Example 7 were carried out in the same manner using the same apparatus, the same amounts of reagents and the same conditions with the exception that 7.11 g (0.071 mol) of cyclohexanol was used in place of t-butanol, thereby obtaining the title product as a transparent liquid with a yield of 80 %.

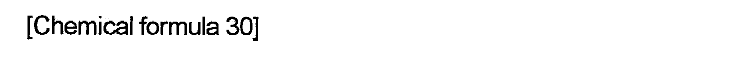

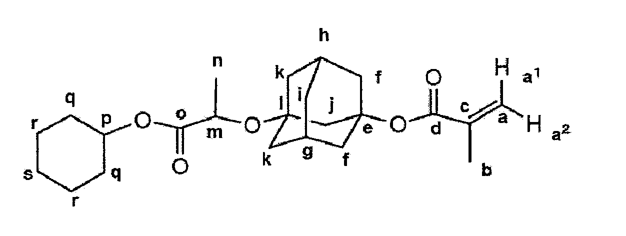

### Nuclear magnetic resonance spectroscopy (NMR), solvent: CDCl₃:

¹H-NMR (500 MHz): 1.24-1.36 (3H), 1.39 (5H, n and r), 1.40 (9H, p), 1.42-1.80 (18H), 1.93 (3H, b), 2.04 (1 H), 3.85 (1 H, m), 3.91 (1 H, p), 5.58 (1 H, a2), 6.15 (1 H, a1)
¹³C-NMR (127 MHz): 17.1 (n), 18.0 (b), 21.3 (g or h), 22.0 (r), 23.3 (g or h), 28.0 (s), 32.2 (q), 38.1 (i), 42.4 (f or k), 43.1 (f or k), 46.0 (j), 62.3 (I), 70.9 (m), 75.4 (e), 75.8 (p), 125 (a), 136.1 (c), 167.2 (d), 170.9 (o)

### Example 15

### Synthesis of 3-{2-[(1,7,7-trimethylbicyclo[2.2.1]hepta-2-yl)oxy]-1-methyl-2-oxoethoxy}-1-adamantyl methacrylate:

The procedures of Example 7 were carried out in the same manner using the same apparatus, the same amounts of reagents and the same conditions with the exception that 10.95 g (0.071 mol) of borneol was used in place of t-butanol, thereby obtaining the title product as a transparent viscous liquid with a yield of 75 %.

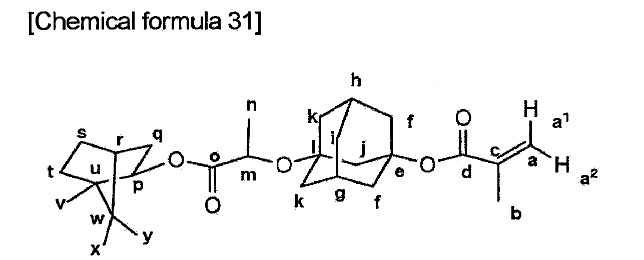

### Nuclear magnetic resonance spectroscopy (NMR), solvent: CDCl₃:

¹H-NMR (500 MHz): 1.11 (6H, x and y), 1.16 (3H, v), 1.24-1.87 (19H), 1.93 (3H, b), 3.85 (1 H, m), 3.89 (1 H, p), 5.58 (1 H, a2), 6.15 (1 H, a1)
¹³C-NMR (127 MHz): 13.5 (v), 17.1 (n), 18.0 (b), 19.5 (x and y), 21.3 (g or h), 22.0 (r), 23.3 (g or h), 23.3 (s), 30.2 (t), 32.5 (q), 32.2 (q), 38.1 (i), 42.4 (f or k), 43.1 (f or k), 45.4 (r), 46.0 (j), 49.5 (u), 50.6 (w), 62.3 (l), 70.9 (m), 75.4 (e), 82.4 (p), 125 (a), 136.1 (c), 167.2 (d), 170.9 (o)

### Example 16

### Synthesis of 3-{1-methyl-2-oxo-2-[(2-oxohexahydro-2H-3,5-methanocyclopenta[b]furan-6-yl)oxy] ethoxy}- 1-adamantyl methacrylate:

The procedures of Example 7 were carried out in the same manner using the same apparatus, the same amounts of reagents and the same conditions with the exception that 10.95 g (0.071 mol) of 5-hydroxy-2,6-norbornanecarbolactone was used in place of t-butanol, thereby obtaining the crude title product. Crystallization was performed in the same manner as in Example 2 to obtain the title product as a white solid with a yield of 62%.

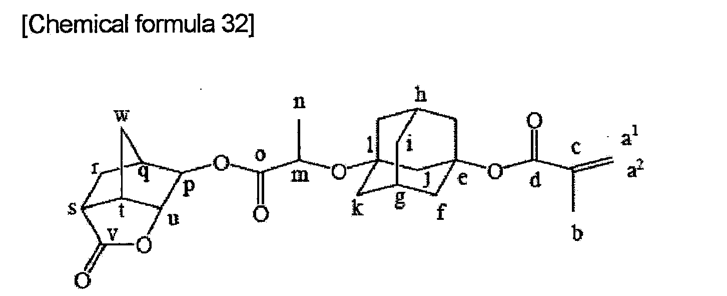

### Nuclear magnetic resonance spectroscopy (NMR), solvent: CDCl₃:

¹H-NMR (500 MHz): 1.24-1.73 (13H), 1.93 (3H, b), 2.04 (1H), 2.31 (1H, p1 or p2), 2.56 (1H, p1 or p2), 3.45 (1H, m), 4.30 (1H, o1 or o2), 4.55 (1H, o1 or o2), 5.58 (1H, a2), 6.15 (1H, a1)
¹³C-NMR (127 MHz): 18.0 (b), 21.3 (g or h), 23.3 (g or h), 38.1 (i), 40.5 (p), 42.4 (f or k), 43.4 (f or k), 46.3 (j), 59.6 (m), 61.6 (l), 75.4 (e), 75.6 (o), 125.2 (a), 136.1 (c), 167.2 (d), 176.1(n)

### Example 17

### Synthesis of 3-[(5-oxotetrahydrofuran-3-yl)oxy] -1-adamantyl methacrylate:

The procedures of Example 2 were carried out in the same manner using the same apparatus, the same amounts of reagents and the same conditions with the exception that β-hydroxy-γ-butyrolactone was used in place of α-hydroxy-γ-butyrolactone and that a reaction temperature of 120°C was used, thereby obtaining the crude title product with a yield of 2.20 g and a purity of 85.1 %. Crystallization was performed in the same manner as in Example 2 to obtain the title product as a white solid with a yield of 1.85 g and a purity of 98.7 %.

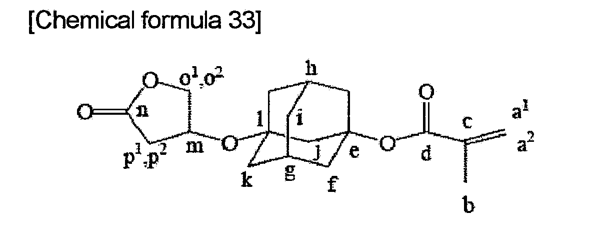

### Nuclear magnetic resonance spectroscopy (NMR), solvent: CDCl₃:

¹ H-NMR (500 MHz): 1.24-1.73 (13H), 1.93 (3H, b), 2.04 (1 H), 2.31 (1 H, p1 or p2), 2.56 (1 H, p1 or p2), 3.45 (1 H, m), 4.30 (1 H, o1 or o2), 4.55 (1 H, o1 or o2), 5.58 (1 H, a2), 6.15 (1 H, a1)
¹³C-NMR (127 MHz): 18.0 (b), 21.3 (g or h), 23.3 (g or h), 38.1 (i), 40.5 (p), 42.4 (f or k), 43.4 (f or k), 46.3 (j), 59.6 (m), 61.6 (l), 75.4 (e), 75.6 (o), 125.2 (a), 136.1 (c), 167.2 (d), 176.1 (n)

### Example 18

### Synthesis of 3-{2-[(2-oxohexahydro-2H-3,5-methanocyclopenta[b]furan-6-yl)oxy]ethoxy}-1-adamantyl methacrylate:

In a 50 mL four-necked, eggplant-shaped flask equipped with a dropping funnel, a stirrer, a condenser and an air inlet tube, 2.80 g (10 mmol) of 3-(2-hydroxyethoxy)-1-adamantyl methacrylate (manufactured by Idemitsu Kosan Co., Ltd.), 1.21 g of triethylamine, 3 mg (1000 ppm by mass) of methoquinone and 20 mL of THF were charged and cooled to 0 ° C with stirring in an ice bath. Then, 1.26 g (11 mmol) of methanesulfonyl chloride was added dropwise to the reactor over 5 minutes. The ice bath was removed 10 minutes after the termination of the dropwise addition. The reaction mixture was then stirred for 30 minutes at room temperature. Thereafter, the dropping funnel and the condenser were removed. THF was distilled off under a reduced pressure. After fitting the condenser to the reactor, 12.33 g (80 mmol) of 5-hydroxy-2,6-norbornanecarbolactone and 2.22 g (12 mmol) of tri-n-butylamine were added thereto. With stirring the reaction mixture was heated to 120°C. The stirring was continued for 4 hours while maintaining the temperature at 120 ° C. It was confirmed by gas chromatography that 3-(2-hydroxyethoxy)-1-adamantyl methacrylate completely disappeared. Extraction and washing were then carried out in the same manner as in Example 1 to obtain a crude title product with a yield of 3.12 g and a purity of 85.5 %. This was homogeneously dissolved in 3 mL of methanol, to which 0.1 mL of distilled water was added. Crystals were precipitated when the resulting solution was cooled to 0°C. The crystals were collected by filtration, and washed with cold distilled water. The solvent was then removed under a reduced pressure to obtain the title product as a white solid (yield: 1.68 g, purity: 98.3 %).

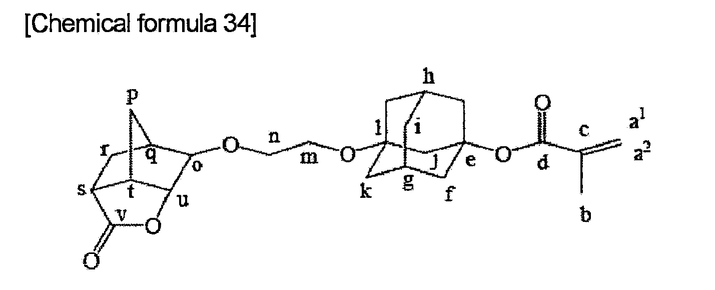

### Nuclear magnetic resonance spectroscopy (NMR), solvent: CDCl₃:

¹H-NMR (500 MHz): 1.24-1.90 (19H, e-1, p, q, r), 1.93 (3H, b), 2.03 (1 H, q), 2.26 (1 H, s), 2.66 (1 H, t), 3.37 (1 H, o), 3.54 (2H, m, n), 4.22 (1 H, u), 5.58 (1 H, a2), 6.15 (1 H, a1)
¹³C-NMR (127 MHz): 18.0 (b), 21.3 (g or h), 23. 3(g or h), 27.2 (r), 30.9 (t), 33.5 (p), 38.1 (i), 41.0 (q), 42.4 (f or k), 43.4 (f or k), 46.3 (j), 46.8 (s), 64.4 (I), 66.4 (m), 69.2 (n), 75.4 (e), 79.0 (o), 84.9 (u), 125.2 (a), 136.1 (c), 167.2 (d), 178.9 (v)

### Example 19

### Synthesis of 3-(2-ethoxy1-methyl-2-oxoethoxy)-1-adamantyl acrylate:

The procedures of Example 3 were carried out in the same manner using the same apparatus, the same amounts of reagents and the same conditions with the exception that 28.54 g (0.095 mol) of 3-methanesulfonyloxy-1-adamantyl acrylate (manufactured by Idemitsu Kosan Co., Ltd) was used in place of 3-methanesulfonyloxy-1-adamantyl methacrylate (manufactured by Idemitsu Kosan Co., Ltd), thereby obtaining the title product with a yield of 86 %.

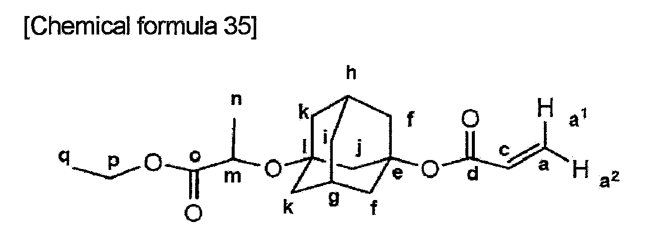

### Nuclear magnetic resonance spectroscopy (NMR), solvent: CDCl₃:

¹H-NMR (500 MHz): 1.24-1.27 (2H), 1.30 (3H, q), 1.36 (3H), 1.39 (5H, n and r), 1.40 (9H, p), 1.42-1.80 (18H), 2.04 (1 H), 3.85 (1 H, m), 3.91 (1 H, p), 4.12 (2H, p), 5.58 (1 H, a2), 6.05 (1H,c),6.15(1H,a1)
¹³C-NMR (127 MHz):14.1 (q), 17.1 (n), 21.3 (g or h), 22.0 (r), 23.3 (g or h), 28.0 (s), 38.1 (i), 42.4 (f or k), 43.1 (f or k), 46.0 (j), 61.6 (p), 62.3 (l), 70.9 (m), 75.4 (e), 125 (a), 128.3 (c), 167.2 (d), 170.9 (o)

### Example 20

### Synthesis of 2-{[3-(acryloyloxy) -1-adamantyl]oxy}propanoic acid:

The procedures of Example 6 were carried out in the same manner using the same apparatus, the same amounts of reagents and the same conditions with the exception that 19.34 g (0.060 mol) of 3-(2-ethoxy-1-methyl-2-oxoethoxy)-1-adamantyl acrylate obtained in Example 19 was used in place of 3-(2-ethoxy-1-methyl-2-oxoethoxy)-1-adamantyl methacrylate obtained in Example 3, thereby obtaining the title product with a yield of 82%.

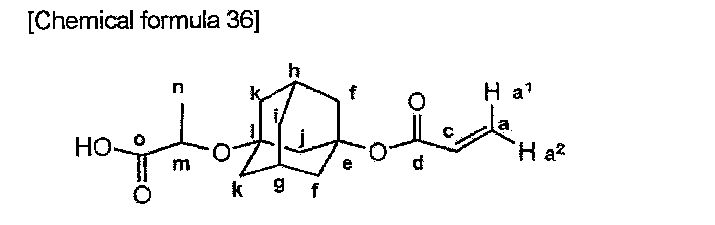

### Nuclear magnetic resonance spectroscopy (NMR), solvent: CDCl₃:

¹H-NMR (500 MHz): 1.24-1.30 (2H), 1.36 (1 H), 1.39 (3H, n), 1.42-1.73 (10H), 2.04 (1 H), 3.85 (1 H, m), 5.58 (1 H, a2), 6.05 (1 H, c), 6.15 (1H, a1)
¹³C-NMR (127 MHz): 17.1 (q), 17.1 (n), 21.3 (g or h), 23.3 (g or h), 38.1 (i), 42.4 (f or k), 43.1 (f or k), 46.0 (j), 62.3 (l), 61.6 (p), 70.9 (m), 75.4 (e), 125 (a), 128.3 (c), 167.2 (d), 170.9 (o)

### Example 21

### Synthesis of 3-{2-[(t-butyloxy)oxy]-1-methyl-2-oxoethoxy}-1-adamantyl acrylate:

The procedures of Example 7 were carried out in the same manner using the same apparatus, the same amounts of reagents and the same conditions with the exception that 19.13 g (0.065 mol) of 2-{[3-(acryloyloxy)-1-adamantyl]oxy}propanoic acid obtained in Example 20 was used in place of 2-{[3-(methacryloyloxy) -1-adamantyl]oxy}propanoic acid obtained in Example 6, thereby obtaining the title product as a transparent liquid with a yield of 65 %.

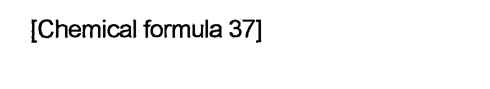

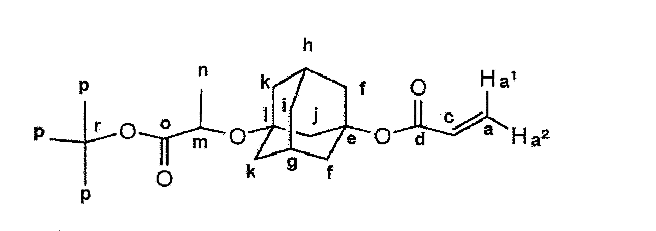

### Nuclear magnetic resonance spectroscopy (NMR), solvent: CDCl₃:

¹H-NMR (500 MHz): 1.24-1.36 (3H), 1.39 (3H, n), 1.40 (9H, p), 1.42-1.73 (10H), 2.04 (1 H), 3.85 (1 H, m), 5.58 (1 H, a2), 6.05 (1 H, c), 6.15 (1 H, a1)
¹³C-NMR (127 MHz): 17.1 (q), 17.1 (n), 21.3 (g or h), 23.3 (g or h), 28.8 (p), 38.1 (i), 42.4 (f or k), 43.1 (f or k), 46.0 (j), 62.3 (I), 61.6 (p), 70.9 (m), 75.4 (e), 82.4 (r), 125 (a), 128.3 (c), 167.2 (d), 170.9 (o)

### Example 22

### Synthesis of 3-{2-[(2-methyl-2-cyclohexyl)oxy]-1-methyl-2-oxoethoxy}-1-adamantyl acrylate:

The procedures of Example 8 were carried out in the same manner using the same apparatus, the same amounts of reagents and the same conditions with the exception that 19.13 g (0.065 mol) of 2-{[3-(acryloyloxy) -1-adamantyl]oxy}propanoic acid obtained in Example 20 was used in place of 2-{[3-(methacryloyloxy) -1-adamantyl]oxy}propanoic acid obtained in Example 6, thereby obtaining the title product as a transparent liquid with a yield of 60 %.

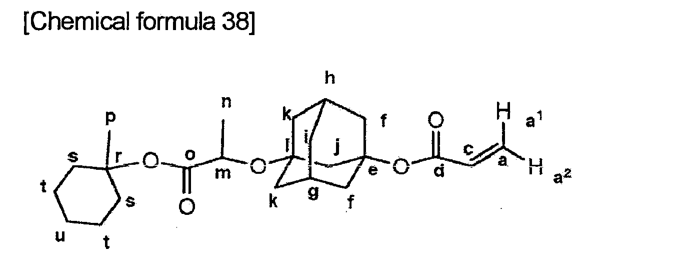

### Nuclear magnetic resonance spectroscopy (NMR), solvent: CDCl₃:

¹H-NMR (500 MHz): 1.24-1.30 (2H), 1.36 (1 H), 1.39 (3H, n), 1.42-1.76 (23H), 2.04 (1 H), 3.85 (1 H, m), 5.58 (1 H, a2), 6.05 (1 H, c), 6.15 (1 H, a1)
¹³C-NMR (127 MHz): 17.1 (q), 17.1 (n), 19.5 (t), 21.3 (g or h), 23.3 (g or h), 24.1 (p), 28.3 (u), 38.1 (i), 40.6 (s), 42.4 (f or k), 43.1 (f or k), 46.0 (j), 62.3 (l), 61.6 (p), 70.9 (m), 75.4 (e), 88.7 (r), 125 (a), 128.3 (c), 167.2 (d), 170.9 (o)

### Example 23

### Synthesis of 3-{2-[(2-ethyl-2-cyclohexyl)oxy]-1-methyl-2-oxoethoxy}-1-adamantyl acrylate:

The procedures of Example 9 were carried out in the same manner using the same apparatus, the same amounts of reagents and the same conditions with the exception that 19.13 g (0.065 mol) of 2-{[3-(acryloyloxy)-1-adamantyl]oxy}propanoic acid obtained in Example 20 was used in place of 2-{[3-(methacryloyloxy) -1-adamantyl]oxy}propanoic acid obtained in Example 6, thereby obtaining the title product as a transparent liquid with a yield of 63 %.

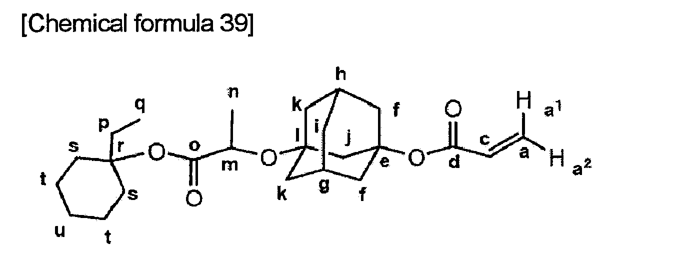

### Nuclear magnetic resonance spectroscopy (NMR), solvent: CDCl₃:

¹H-NMR (500 MHz): 0.96 (3H, q), 1.24-1.30 (2H), 1.36 (1 H), 1.39 (3H, n), 1.42-1.76 (23H), 2.04 (1 H), 3.85 (1 H, m), 5.58 (1 H, a2), 6.05 (1 H, c), 6.15 (1 H, a1)
¹³C-NMR (127 MHz): 5.9 (q), 17.1 (q), 17.1 (n), 19.5 (t), 21.3 (g or h), 23.3 (g or h), 24.1 (p), 28.3 (u), 38.1 (i), 40.6 (s), 42.4 (f or k), 43.1 (f or k), 46.0 (j), 62.3 (l), 61.6 (p), 70.9 (m), 75.4 (e), 88.7 (r), 125 (a), 128.3 (c), 167.2 (d), 170.9 (o)

### Example 24

### Synthesis of 3-{2-[(cyclohexyloxy)methoxy]-1-methyl-2-oxoethoxy}-1-adamantyl acrylate:

The procedures of Example 10 were carried out in the same manner using the same apparatus, the same amounts of reagents and the same conditions with the exception that 19.13 g (0.065 mol) of 2-{[3-(acryloyloxy) -1-adamantyl]oxy}propanoic acid obtained in Example 20 was used in place of 2-{[3-(methacryloyloxy) -1-adamantyl]oxy}propanoic acid obtained in Example 6, thereby obtaining the title product with a yield of 60 %.

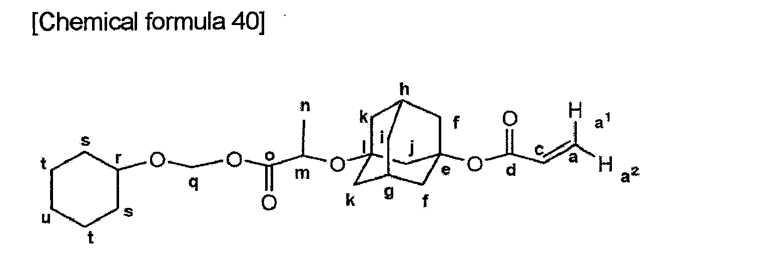

### Nuclear magnetic resonance spectroscopy (NMR), solvent: CDCl₃:

¹H-NMR (500 MHz): 1.24-1.30 (2H), 1.36 (1H), 1.38 (3H, n), 1.39-1.73 (20H), 2.04 (1 H), 2.79 (1 H, r), 3.85 (1 H, m), 5.58 (1 H, a2), 6.15 (1 H, a1), 6.05 (1 H, c), 6.16 (2H, q)
¹³C-NMR (127 MHz): 17.1 (q), 17.1 (n), 18.0 (b), 21.3 (g or h), 22.1 (t), 23.3 (g or h), 28.3 (u), 34.0 (s), 38.1 (i), 42.4 (f or k), 43.1 (f or k), 46.0 (j), 62.3 (1), 61.6 (p), 70.9 (m), 75.4 (e), 78.5 (r), 91.2 (q), 125 (a), 128.3 (c), 167.2 (d), 170.9 (o)

### Example 25

### Synthesis of 3-{2[(2-ethyl-2-cydopentyl)oxy]-1-methyl-2-oxoethoxy}-1-adamantyl acrylate:

The procedures of Example 11 were carried out in the same manner using the same apparatus, the same amounts of reagents and the same conditions with the exception that 19.13 g (0.065 mol) of 2-{[3-(acryloyloxy) -1-adamantyl]oxy}propanoic acid obtained in Example 20 was used in place of 2-{[3-(methacryloyloxy) -1-adamantyl]oxy}propanoic acid obtained in Example 6, thereby obtaining the title product as a transparent liquid with a yield of 80%.

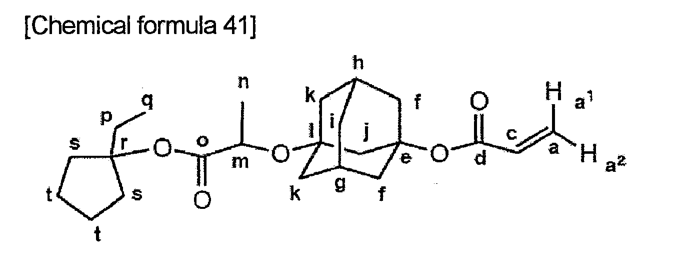

### Nuclear magnetic resonance spectroscopy (NMR), solvent: CDCl₃:

¹H-NMR (500 MHz): 0.96 (3H, q), 1.24-1.30 (2H), 1.36 (1 H), 1.39 (3H, n), 1.42-1.84 (18H), 2.04 (1 H), 3.85 (1 H, m), 5.58 (1 H, a2), 6.05 (1 H, c), 6.15 (1 H, a1)
¹³C-NMR (127 MHz): 5.9 (q), 17.1 (q), 17.1 (n), 21.3 (g or h), 22.2 (t), 23.3 (g or h), 28.3 (u), 31.6 (p), 38.1 (i), 39.6 (s), 42.4 (f or k), 43.1 (f or k), 46.0 (j), 62.3 (l), 61.6 (p), 70.9 (m), 75.4 (e), 88.7 (r), 125 (a), 128.3 (c), 167.2 (d), 170.9 (o)

### Example 26

### Synthesis of 3-{2-[(2-methyl-2-adamantyl)oxy]-1-methyl-2-oxoethoxy}-1-adamantyl acrylate:

The procedures of Example 12 were carried out in the same manner using the same apparatus, the same amounts of reagents and the same conditions with the exception that 19.13 g (0.065 mol) of 2-{[3-(acryloyloxy) -1-adamantyl]oxy}propanoic acid obtained in Example 20 was used in place of 2-{[3-(methacryloyloxy) -1-adamantyl]oxy}propanoic acid obtained in Example 6, thereby obtaining the title product as a transparent liquid with a yield of 55 %.

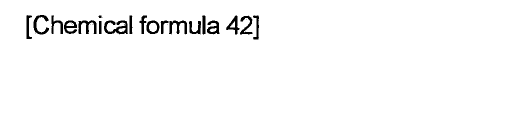

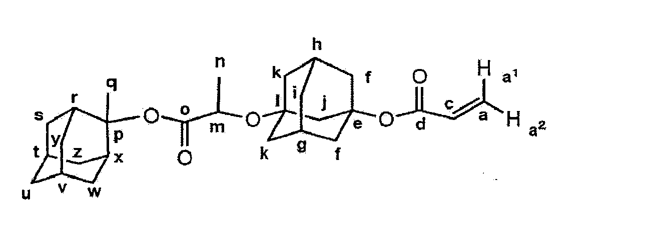

### Nuclear magnetic resonance spectroscopy (NMR), solvent: CDCl₃:

¹H-NMR (500 MHz): 1.18 (5H), 1.36 (1 H), 1.38 (3H, n), 1.39-1.91 (23H), 2.04 (1 H), 3.85 (1 H, m), 5.58 (1 H, a2), 6.05 (1 H, c), 6.15 (1 H, a1)
¹³C-NMR (127 MHz): 17.1 (n), 19.4 (q), 21.3 (g or h), 22.2 (t), 23.3 (g or h), 28.3 (u), 29.3 (s, y, v or w), 31.5 (t or v), 31.6 (p), 37.8 (u), 38.1 (i), 39.6 (s), 40.0 (r or x), 42.4 (f or k), 43.1 (f or k), 46.0 (j), 62.3 (I), 70.9 (m), 75.4 (e), 83.4 (p), 88.7 (r), 125 (a), 128.3 (c), 167.2 (d), 170.9 (o)

### Example 27

### Synthesis of 3-{2-[(2-ethyl-2-adamantyl)oxy]-1-methyl-2-oxoethoxy}-1-adamantyl acrylate:

The procedures of Example 13 were carried out in the same manner using the same apparatus, the same amounts of reagents and the same conditions with the exception that 19.13 g (0.065 mol) of 2-{[3-(acryloyloxy) -1-adamantyl]oxy}propanoic acid obtained in Example 20 was used in place of 2-{[3-(methacryloyloxy) -1-adamantyl]oxy}propanoic acid obtained in Example 6, thereby obtaining the title product as a transparent liquid with a yield of 80 %.

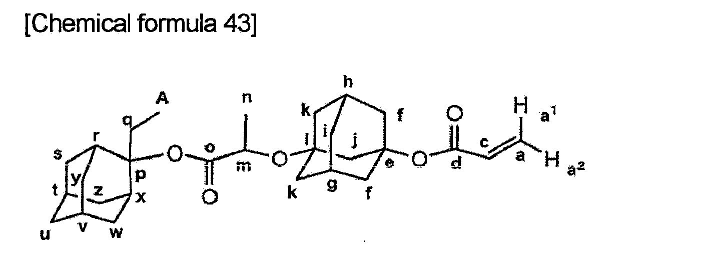

### Nuclear magnetic resonance spectroscopy (NMR), solvent: CDCl₃:

¹H-NMR (500 MHz): 0.96 (3H, A), 1.18 (5H), 1.36 (1H), 1.38 (3H, n), 1.39-1.91 (23H), 1.93 (3H, b), 2.04 (1H), 3.85 (1H, m), 5.58 (1H, a2), 6.05 (1H, c), 6.15 (1H, a1)
¹³C-NMR (127 MHz): 6.5 (A), 17.1 (n), 21.3 (g or h), 22.2 (t), 23.3 (g or h), 27.2 (q), 28.3 (u), 29.3 (s, y, v or w), 31.5 (t or v), 31.6 (p), 37.8 (u), 38.1 (i), 39.6 (s), 40.0 (r or x), 42.4 (f or k), 43.1 (f or k), 46.0 (j), 62.3 (l), 70.9 (m), 75.4 (e), 83.4 (p), 88.7 (r), 125 (a), 128.3 (c), 167.2 (d), 170.9 (o)

### Example 28

### Synthesis of 3-{2-[(cyclohexyl)oxy]-1-methyl-2-oxoethoxy}-1-adamantyl acrylate:

The procedures of Example 14 were carried out in the same manner using the same apparatus, the same amounts of reagents and the same conditions with the exception that 19.13 g (0.065 mol) of 2-{[3-(acryloyloxy) -1-adamantyl]oxy}propanoic acid obtained in Example 20 was used in place of 2-{[3-(methacryloyloxy) -1-adamantyl]oxy}propanoic acid obtained in Example 6, thereby obtaining the title product as a transparent viscous liquid with a yield of 86 %.

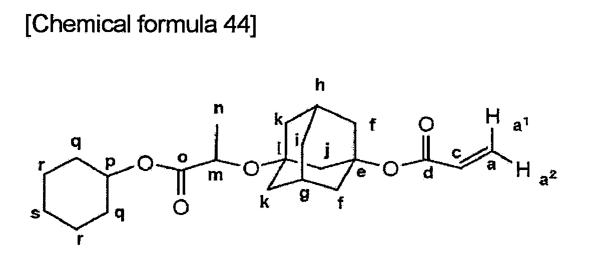

### Nuclear magnetic resonance spectroscopy (NMR), solvent: CDCl₃:

¹H-NMR (500 MHz): 1.24-1.36 (3H), 1.39 (5H, n and r), 1.40 (9H, p), 1.42-1.80 (18H), 2.04 (1H), 3.85 (1H, m), 3.91 (1H, p), 5.58 (1H, a2), 6.05 (1H, c), 6.15 (1H, a1)
¹³C-NMR (127 MHz): 17.1 (n), 21.3 (g or h), 22.0 (r), 23.3 (g or h), 28.0 (s), 32.2 (q), 38.1 (i), 42.4 (f or k), 43.1 (f or k), 46.0 (j), 62.3 (I), 70.9 (m), 75.4 (e), 75.8 (p), 125 (a), 128.3 (c),167.2 (d), 170.9 (o)

### Example 29

### Synthesis of 3-{2-[(1,7,7-trimethylbicyclo[2.2.1]hepta-2-yl)oxy]-1-methyl-2-oxoethoxy}-1-adamantyl acrylate:

The procedures of Example 15 were carried out in the same manner using the same apparatus, the same amounts of reagents and the same conditions with the exception that 19.13 g (0.065 mol) of 2-{[3-(acryloyloxy) -1-adamantyl]oxy}propanoic acid obtained in Example 20 was used in place of 2-{[3-(methacryloyloxy) -1-adamantyl]oxy}propanoic acid obtained in Example 6, thereby obtaining the title product as a transparent viscous liquid with a yield of 76 %.

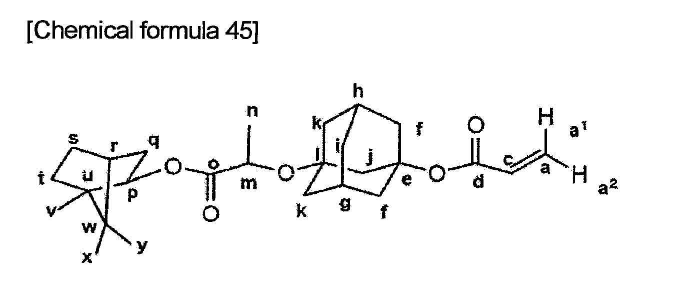

### Nuclear magnetic resonance spectroscopy (NMR), solvent: CDCl₃:

¹H-NMR (500 MHz): 1.11 (6H, x and y), 1.16 (3H, v), 1.24-1.87 (19H), 1.93 (3H, b), 3.85 (1H, m), 3.89 (1H, p), 5.58 (1H, a2), 6.05 (1H, c), 6.15 (1H, a1)
¹³C-NMR (127 MHz): 13.5 (v), 17.1 (n), 18.0 (b), 19.5 (x and y), 21.3 (g or h), 22.0 (r), 23.3 (g or h), 23.3 (s), 30.2 (t), 32.5 (q), 32.2 (q), 38.1 (i), 42.4 (f or k), 43.1 (f or k), 45.4 (r), 46.0 (j), 49.5 (u), 50.6 (w), 62.3 (l), 70.9 (m), 75.4 (e), 82.4 (p), 125 (a), 283.3 (c), 167.2 (d), 170.9 (o)

### Example 30

### Synthesis of 3-{1-methyl-2-oxo-2-[(2-oxohexahydro-2H-3,5-methanocyclopenta[b]furan-6-yl)oxy] ethoxy}-1-adamantyl acrylate:

The procedures of Example 16 were carried out in the same manner using the same apparatus, the same amounts of reagents and the same conditions with the exception that 19.13 g (0.065 mol) of 2-{[3-(acryloyloxy) -1-adamantyl]oxy}propanoic acid obtained in Example 20 was used in place of 2-{[3-(methacryloyloxy)-1-adamantyl]oxy}propanoic acid obtained in Example 6, thereby obtaining the title product as a white solid with a yield of 58 %.

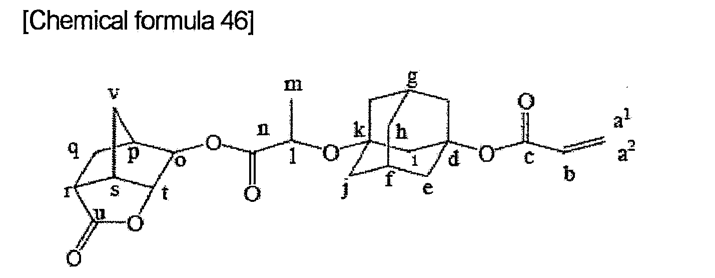

### Nuclear magnetic resonance spectroscopy (NMR), solvent: CDCl₃:

¹H-NMR (500 MHz): 1.24-1.88 (21H, d-k, m, q, v), 2.03 (1H, p), 2.26 (1 H, r), 2.66 (1H, s), 3.85 (1 H, I), 4.49 (1 H, o or t), 4.50 (1 H, o or t), 6.05 (1 H, b), 5.80 (1 H, a2), 6.43 (1 H, a1) ¹³C-NMR (127 MHz): 17.1 (m), 21.3 (f or g), 23.3 (f or g), 27.1 (q), 30.8 (s), 33.4 (v), 38.1 (h), 39.9 (p), 42.4 (e or j), 43.1 (e or j), 46.0 (i), 46.5 (r), 61.3 (k), 71.2 (I),75.1 (d), 81.6 (o), 83.8 (t), 128.3 (b), 130.2 (a), 166.5 (c), 170.9 (n), 178.9 (u)

### Example 31

### Synthesis of 3-[(2,6-norbornanecarbolactone-5-yl)oxy]-1-adamantyl acrylate:

The procedures of Example 1 were carried out in the same manner using the same apparatus, the same amounts of reagents and the same conditions with the exception that 3.00 g (10 mmol) of 3-methanesulfonyloxy-1-adamantyl acrylate (manufactured by Idemitsu Kosan Co., Ltd) was used in place of 3-methanesulfonyloxy-1-adamantyl methacrylate (manufactured by Idemitsu Kosan Co., Ltd), thereby obtaining the crude title product with a yield of 3.02 g and a purity of 92.5 %. Crystallization was performed in the same manner as in Example 1 to obtain the title product as a white solid with a yield of 1.72 g and a purity of 99.7 %.

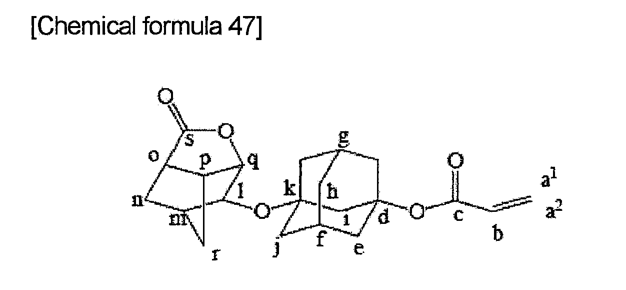

### Nuclear magnetic resonance spectroscopy (NMR), solvent: CDCl₃:

¹H-NMR (500 MHz): 1.24-1.88 (19H, d-k, m, n, r), 2.26 (1H, o), 2.66 (1H, p), 3.37 (1H, l), 4.22 (1H, q), 5.80 (1H, a2), 6.05 (1H, b), 6.43 (1H, a1)
¹³C-NMR (127 MHz): 21.3 (f or g), 23.3 (f or g), 27.2 (n), 30.9 (p), 33.5 (r), 38.1 (h), 41.0 (q), 41.6 (m), 42.4 (e or j), 43.4 (e or j), 46.3 (i), 46.8 (o), 62.5 (k), 74.6 (l), 75.1 (d), 79.0 (o), 84:9 (u), 128.3 (b), 130.2 (a), 166.5 (c), 178.9 (s)

### Example 32

### Synthesis of 3-[(2-oxotetrahydrofuran-3-yl)oxy]-1-adamantyl acrylate:

The procedures of Example 2 were carried out in the same manner using the same apparatus, the same amounts of reagents and the same conditions with the exception that 3.00 g (10 mmol) of 3-methanesulfonyloxy-1-adamantyl acrylate (manufactured by Idemitsu Kosan Co., Ltd) was used in place of 3-methanesulfonyloxy-1-adamantyl methacrylate (manufactured by Idemitsu Kosan Co., Ltd), thereby obtaining the crude title product with a yield of 2.01 g and a purity of 80.9 %. Crystallization was performed in the same manner as in Example 2 to obtain the title product as a white solid with a yield of 1.42 g and a purity of 98.9 %.

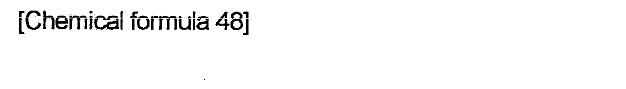

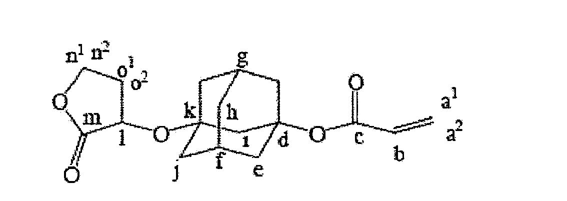

### Nuclear magnetic resonance spectroscopy (NMR), solvent: CDCl₃:

¹H-NMR (500 MHz): 1.24-1.88 (14H, d-k), 2.11 (1 H, o1 or o2), 2.36 (1 H, o1 or o2), 3.65 (1 H, l), 4.25 (1 H, n1 or n2), 4.35 (1 H, n1 or n2), 5.80 (1 H, a2), 6.05 (1 H, b), 6.43 (1 H, a1) ¹³C-NMR (127 MHz): 21.3 (f or g), 23.3 (f or g), 23.9 (o), 38.1 (h), 42.4 (e or j), 43.1 (e or j), 46.0 (i), 61.6 (k), 65.0 (n), 75.1 (d), 85.8 (l), 128.3 (b), 130.2 (a), 166.5 (c), 173.7 (m)

### Example 33

### Synthesis of 3-[(5-oxotetrahydrofuran-3-yl)oxy]-1-adamantyl acrylate:

The procedures of Example 17 were carried out in the same manner using the same apparatus, the same amounts of reagents and the same conditions with the exception that 3.00 g (10 mmol) of 3-methanesulfonyloxy-1-adamantyl acrylate (manufactured by Idemitsu Kosan Co., Ltd) was used in place of 3-methanesulfonyloxy-1-adamantyl methacrylate (manufactured by Idemitsu Kosan Co., Ltd), thereby obtaining the crude title product with a yield of 2.01 g and a purity of 80.9 %. Crystallization was performed in the same manner as in Example 2 to obtain the title product as a white solid with a yield of 1.57 g and a purity of 99.4 %.

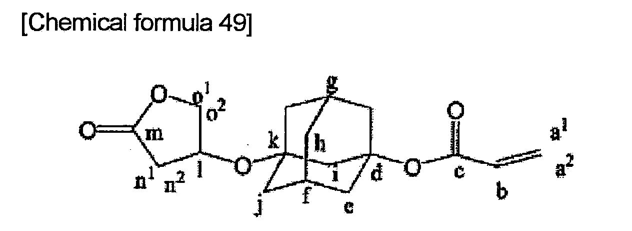

### Nuclear magnetic resonance spectroscopy (NMR), solvent: CDCl₃:

¹H-NMR (500 MHz): 1.24-1.88 (14H, d-k), 2.31 (1H, n1 or n2), 2.56 (1H, n1 or n2), 3.45 (1 H, l), 4.30 (1 H, o1 or o2), 4.55 (1 H, o1 or o2), 5.80 (1 H, a2), 6.05 (1 H, b), 6.43 (1 H, a1) ¹³C-NMR (127 MHz): 21.3 (f or g), 23.3 (f or g), 38.1 (h), 40.5 (n), 42.4 (e or j), 43.4 (e or j), 46.3 (i), 59.6 (l), 61.6 (k), 75.1 (d), 75.6 (o), 128.3 (b), 130.2 (a), 166.5 (c), 176.1 (m)

### Example 34

### Synthesis of 3-{2-[(2-oxohexahydro-2H-3,5-methanocyclopenta[b]furan-6-yl)oxy]ethoxy}-1-adamantyl acrylate:

The procedures of Example 18 were carried out in the same manner using the same apparatus, the same amounts of reagents and the same conditions with the exception that 2.66 g (10 mmol) of 3-(1-hydroxyethoxy)-1-adamantyl acrylate (manufactured by Idemitsu Kosan Co., Ltd) was used in place of 3-(2-hydroxyethoxy)-1-adamantyl methacrylate (manufactured by Idemitsu Kosan Co., Ltd), thereby obtaining the crude title product with a yield of 3.15 g and a purity of 89.9 %. Crystallization was performed in the same manner as in Example 18 to obtain the title product as a white solid with a yield of 1.72 g and a purity of 99.7%.

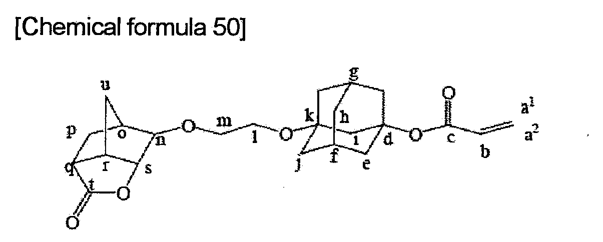

### Nuclear magnetic resonance spectroscopy (NMR), solvent: CDCl₃:

¹H-NMR (500 MHz): 1.24-1.89 (19H, d-k, o, p, u), 2.26 (1 H, q), 2.66 (1 H, r),3.37 (1 H, n), 3.54 (1 H, I or m), 3.55 (1 H, I or m), 6.05 (1 H, b), 5.80 (a2), 6.43 (1 H, a1)
¹³C-NMR (127 MHz): 21.3 (f or g), 23.3 (f or g), 27.2 (p), 30.9 (r), 33.5 (u), 38.1 (h), 41.0 (o), 42.4 (e or j), 43.1 (e or j), 46.0 (i), 46.8 (q), 64.4 (k), 66.4 (l), 69.2 (m), 75.1 (d), 79.0 (n), 84.9 (s), 128.3 (b), 130.2 (a), 166.5 (c), 178.9 (t)

### Example 35

The procedures of Example 5 were carried out in the same manner using the same apparatus, the same amounts of reagents and the same conditions with the exception that 10.46 g (25 mmol) of 3-{2-[(2-ethyl-2-cyclohexyl)oxy]-1-methyl-2-oxoethoxy}-1-adamantyl methacrylate obtained in Example 9 was used as a monomer in place of 3-[(2,6-norbornanecarbolactone- 5-yl)oxy]-1-adamantyl methacrylate obtained in Example 1, thereby obtaining 12.65 g of a polymer. The polymer was measured for its molecular weight by a GPC method to reveal that the polystyrene equivalent molecular weight was 10,300 and Mw/Mn was 2.6.

### Example 36

The procedures of Example 5 were carried out in the same manner using the same apparatus, the same amounts of reagents and the same conditions with the exception that 4.25 g (25 mmol) of 2-oxotetrahydrofuran-3-yl methacrylate was used as a monomer in place of Adamantate HM (manufactured by Idemitsu Kosan Co., Ltd.), thereby obtaining 8.21 g of a polymer. The polymer was measured for its molecular weight by a GPC method to reveal that the polystyrene equivalent molecular weight was 9,800 and Mw/Mn was 2.3.

### Example 37

In 200 mL of 2-butanone was dissolved 18.62 g (50 mmol) of 3-[(2,6-norbornanecarbolactone-5-yl)oxy]-1-adamantyl methacrylate obtained in Example 1 as a monomer, to which 1.15 g (5 mmol) of V601 (manufactured by Wako Pure Chemical Industries Ltd.) as a polymerization initiator. The mixture was subjected three times to freezing deaeration in the atmosphere of nitrogen at a temperature of liquid nitrogen, followed by reaction under reflux conditions for 6 hours in the atmosphere of nitrogen. The reaction was terminated by addition of 20 mL of methanol to the reaction liquid. This was
poured dropwise into 3,000 L of methanol with stirring for reprecipitation. The precipitate was filtered on a glass filter and dried at 60°C for 3 days to obtain 14.59 g of a polymer. The polymer was measured for its molecular weight by a GPC method to reveal that the polystyrene equivalent molecular weight was 11,300 and Mw/Mn was 3.8.

### Example 38

The procedures of Example 37 were carried out in the same manner using the same apparatus, the same amounts of reagents and the same conditions with the exception that 16.82 g (50 mmol) of 3-(2-ethoxy-1-methyl-2-oxoethoxy)- 1-adamantyl methacrylate obtained in Example 3 was used as a monomer, thereby obtaining 14.65 g of a polymer. The polymer was measured for its molecular weight by a GPC method to reveal that the polystyrene equivalent molecular weight was 10,300 and Mw/Mn was 2.6.

### Example 39

The procedures of Example 37 were carried out in the same manner using the same apparatus, the same amounts of reagents and the same conditions with the exception that 19.52 g (50 mmol) of 3-{2-[(cyclohexyl)oxy]-1-methyl-2-oxoethoxy}-1-adamantyl methacrylate obtained in Example 14 was used as a monomer, thereby obtaining 12.65 g of a polymer. The polymer was measured for its molecular weight by a GPC method to reveal that the polystyrene equivalent molecular weight was 13,200 and Mw/Mn was 3.1.

### Comparative Example 1

The procedures of Example 37 were carried out in the same manner using the same apparatus, the same amounts of reagents and the same conditions with the exception that 8.50 g (50 mmol) of 2- oxotetrahydrofuran-3-yl methacrylate was used as a monomer, thereby obtaining 6.57 g of a polymer. The polymer was measured for its molecular weight by a GPC method to reveal that the polystyrene equivalent molecular weight was 8,600 and Mw/Mn was 2.4.

### Comparative Example 2

The procedures of Example 37 were carried out in the same manner using the same apparatus, the same amounts of reagents and the same conditions with the exception that 11.11 g (50 mmol) of 2,6-norbomanecarbolactone-5-yl methacrylate was used as a monomer, thereby obtaining 9.85 g of a polymer. The polymer was measured for its molecular weight by a GPC method to reveal that the polystyrene equivalent molecular weight was 12,300 and Mw/Mn was 2.7.

### Example 40

Each of the polymers obtained in Example 37 and Comparative Examples 1 and 2 was dissolved in an amount of 4.2 g in 23.8 g of 1,4-dioxane and 7 g of y-butyrolactone. Each of the resulting solutions was spin coated on a silicon wafer of 2 inches. The thickness of each film (initial film thickness) was measured with a critical dimension scanning microscope (CD-SEM). The results are given in Table 1. Each wafer was then subjected to a reactive ion etching (RIE) treatment. The RIE treatment was performed using CF₄ and H₂ gases at 200 W and 10 MPa for 30 minutes. The thickness of each film after the RIE treatment (film thickness after treatment) was measured with a CD-SEM. The results are given in Table 1.
An etching rate was calculated by dividing the amount of reduction of the film thickness by the RIE treatment time. It is revealed that the etching rate of the polymer obtained in Example 37 is best.

[Table 1]

**Table 1**

| Polymer | Initial film thickness (nm) | Film thickness after treatment (nm) | Reduction of film thickness (nm) | Etching rate (nm/min) |
|---|---|---|---|---|
| Polymer of Example 37 | 969.8 | 518.9 | 450.9 | 15.0 |
| Polymer of Comparative Example 1 | 745.0 | 0 | 745.0 | - |
| Polymer of Comparative Example 2 | 828.8 | 297.0 | 531.8 | 17.7 |

### [Industrially Applicability]

The polymer composed of the polymerizable compound of the present invention having an alicyclic structure and a terminal group has high dry-etching resistance and is highly transparent to a short wavelength light and, therefore, is suited as a film forming component for a photoresist composition for use with a far ultraviolet light, particularly an ArF excimer laser beam or an F₂ excimer laser beam.

## Claims

1. A polymerizable compound having an alicyclic structure and a polymerizable group and represented by the general formula (1): 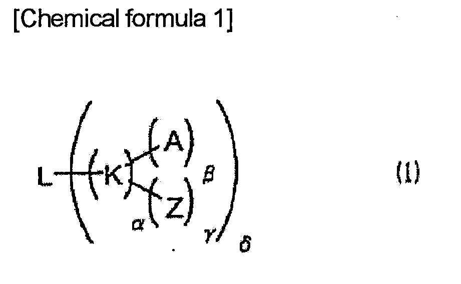 wherein
A represents the polymerizable group selected from those represented by the following formulas (2), (3) and (4): 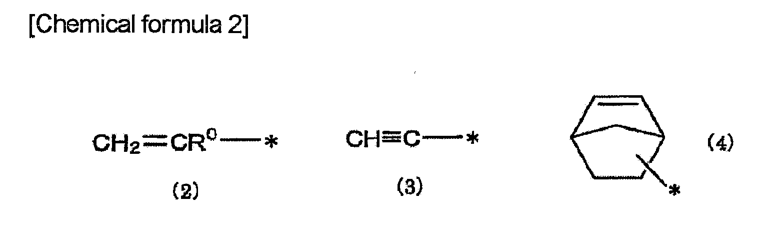 (wherein R⁰ represents a hydrogen atom, a fluorine atom, a methyl group, an ethyl group or a trifluoromethyl group)
with the proviso that when there are a plurality of such A groups, they may be the same or different;
K represents a linking group selected from those represented by the following formulas (5), (6), (7), (8) and (9): 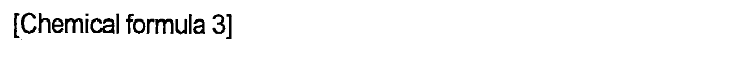 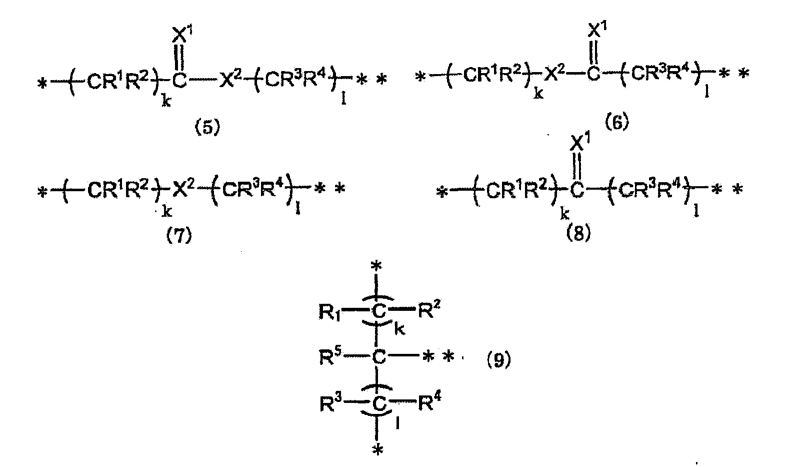 (wherein R¹, R², R³, R⁴ and R⁵ each represent a hydrogen atom, a C₁ to C₁₀ alkyl group which may contain a hetero atom, or a halogen atom, k and l each represent an integer of 0 to 10, X¹ and X² each represent an oxygen atom, a sulfur atom or an NH group, * indicates the bond to the polymerizable group or a terminal group, and ** indicates the bond to an alicyclic group)
with the proviso that when there are a plurality of such K groups, they may be the same or different;
L represents the alicyclic group selected from those represented by the following general formulas (10), (11), (12), (13), (14), (15) and (16): 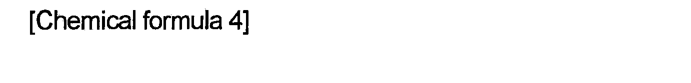 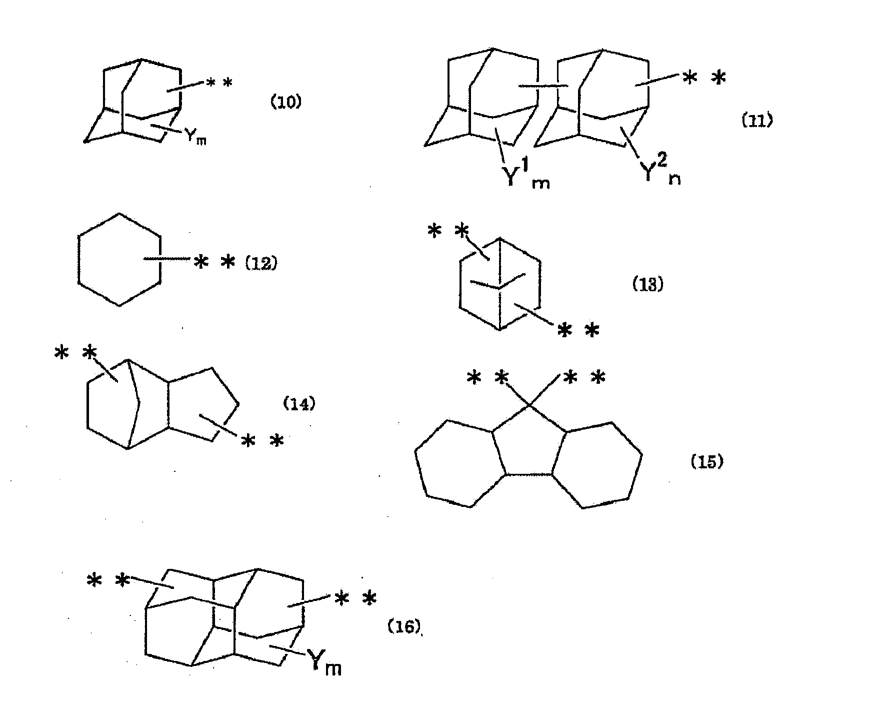 (wherein Y, Y¹ and Y² each represent a hydrogen atom, a C₁ to C₁₀ alkyl group, a halogen atom, a hydroxyl group, a mercapto group or a methylcyano group, or two Ys, two Y¹s and two Y²s are each taken together to represent =O or =S, with the proviso that when there are a plurality of such Y groups, a plurality of such Y¹ groups and a plurality of such Y² groups, they may be the same or different, respectively, and m and n each represent an integer of 0 to 15);
Z represents the terminal group selected from those represented by the following formulas (17) and (18): 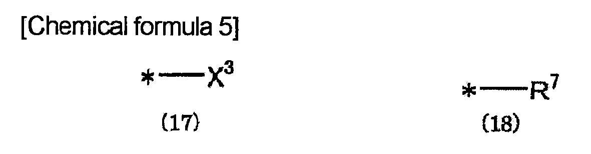 (wherein R⁷ represents a C₁ to C₃₀ alkyl group or a C₅ to C₃₀ cycloalkyl group each of which may contain a hetero atom, a hydroxyl group, a mercapto group, an ether group, a thioether group, a cyano group, a ketone group, a thioketone group, a ketal group, a thioketal group, an acetal group, a thioacetal group, a lactone group, a thiolactone group, a carbonate group, a thiocarbonate group, an amine group, an amide group, an alkylsulfonyl group, an ester group or a thioester group, and X³ represents a hydrogen atom, a hydroxyl group, a mercapto group, an amino group, PO₃, a nitro group, OSO₂CH₃, SSO₂CH₃ or NHSO₂CH₃)
with the proviso that when there are a plurality of such Z groups, they may be the same or different; and
α is an integer of 1 or more, β and γ are each an integer of 0 or more and δ is an integer of 1 to 16, with the proviso that the polymerizable compound contains at least one A group and at least one Z group.

2. The polymerizable compound as recited in claim 1, wherein L in the general formula (1) is a group represented by the formula (10) or (11).

3. The polymerizable compound as recited in claim 1, wherein at least one of K in the general formula (1) is a group selected from those represented by the formula (5) and (7).

4. A polymerizable compound having an alicyclic structure, a polymerizable group and a terminal group and represented by the general formula (19): 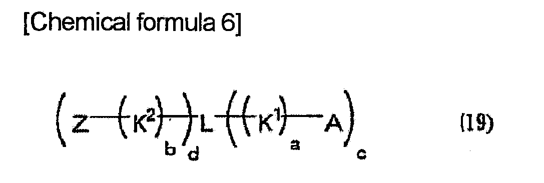 wherein
A represents the polymerizable group selected from those represented by the formulas (2), (3) and (4): 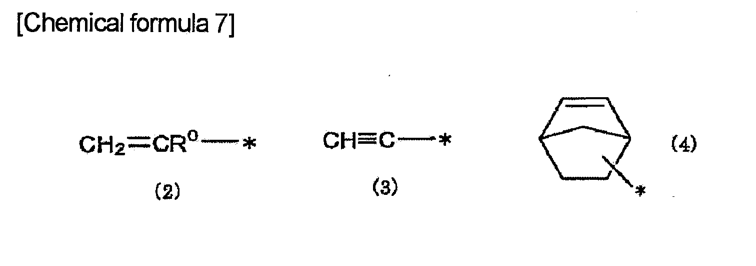 (wherein R⁰ represents a hydrogen atom, a fluorine atom, a methyl group, an ethyl group or a trifluoromethyl group)
with the proviso that when there are a plurality of such A groups, they may be the same or different;
K¹ and K² each represent a linking group selected from those represented by the following formulas (5), (6), (7), (8) and (9): 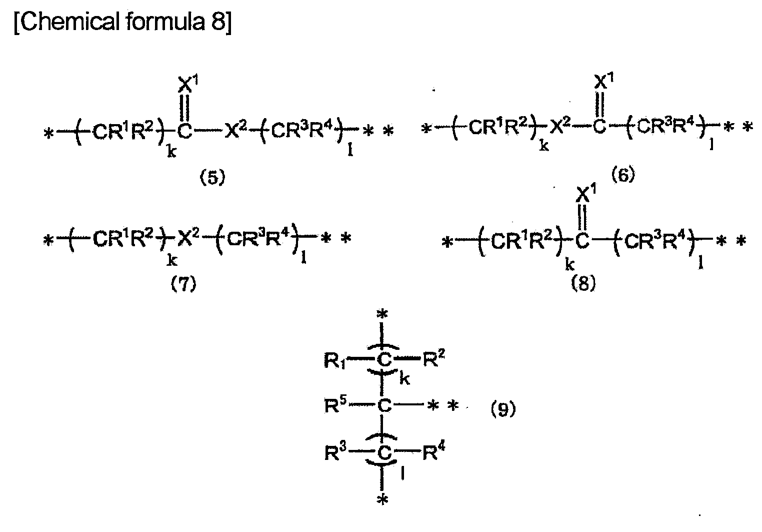 (wherein R¹, R², R³, R⁴ and R⁵ each represent a hydrogen atom, a C₁ to C₁₀ alkyl group which may contain a hetero atom, or a halogen atom, k and l each represent an integer of 0 to 10, X¹ and X² each represent an oxygen atom, a sulfur atom or an NH group, * indicates the bond to the polymerizable group or the terminal group, and ** indicates the bond to an alicyclic group)
with the proviso that when there are a plurality of such K¹ and K² groups, they may be the same or different;
L represents the alicyclic group selected from those represented by the following general formulas (10), (11), (12), (13), (14), (15) and (16): 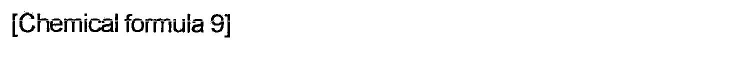 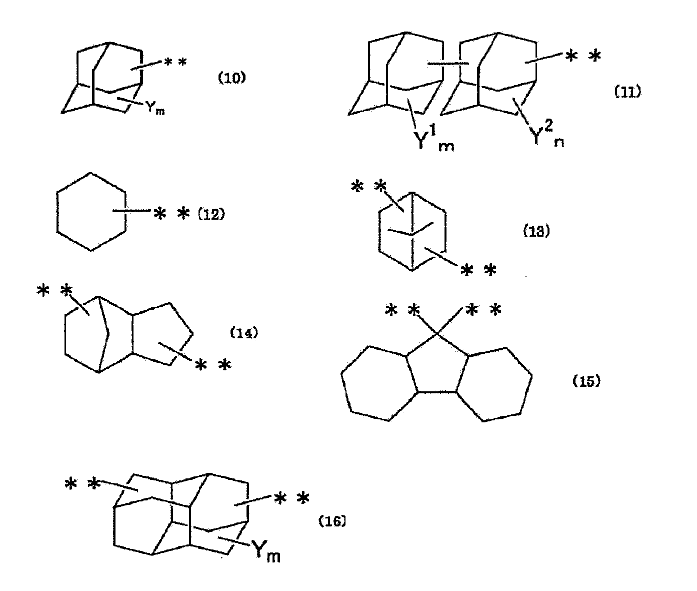 (wherein Y, Y¹ and Y² each represent a hydrogen atom, a C₁ to C₁₀ alkyl group, a halogen atom, a hydroxyl group, a mercapto group or a methylcyano group, or two Ys, two Y¹s and two Y²s are each taken together to represent =O or =S, with the proviso that when there are a plurality of such Y groups, a plurality of such Y¹ groups and a plurality of such Y² groups, they may be the same or different, respectively, and m and n each represent an integer of 0 to 15);
Z represents the terminal group selected from those represented by the following formulas (17) and (18): 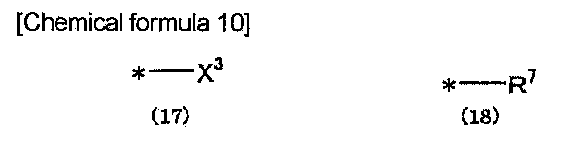 (wherein R⁷ represents a C₁ to C₃₀ alkyl group or a C₅ to C₃₀ cycloalkyl group each of which may contain a hetero atom, a hydroxyl group, a mercapto group, an ether group, a thioether group, a cyano group, a ketone group, a thioketone group, a ketal group, a thioketal group, an acetal group, a thioacetal group, a lactone group, a thiolactone group, a carbonate group, a thiocarbonate group, an amine group, an amide group, an alkylsulfonyl group, an ester group or a thioester group, and X³ represents a hydrogen atom, a hydroxyl group, a mercapto group, an amino group, PO₃, a nitro group, OSO₂CH₃, SSO₂CH₃ or NHSO₂CH₃)
with the proviso that when there are a plurality of such Z groups, they may be the same or different; and
a and b are each an integer of 1 or more, c and d are each an integer of 1 to 15, with the proviso that c+d ≤ 16.

5. The polymerizable compound as recited in claim 4, wherein, in the general formula (19), A is a group represented by the formula (2), and at least one K¹ is a group represented by the formula (5) with the proviso that k is 0.

6. The polymerizable compound as recited in claim 4, wherein, in the general formula (19), A is a group represented by the formula (2), at least one K¹ is a group represented by the formula (5) with the proviso that k is 0, and at least one K² is a group represented by the formula (7) with the proviso that I is 0.

7. The polymerizable compound as recited in claim 6, wherein, in the general formula (19), A is a group represented by the formula (2), at least one K¹ is a group represented by the formula (5) with the proviso that X¹ and X² are each an oxygen atom and that k is 0, and at least one K² is a group represented by the formula (7).

8. The polymerizable compound as recited in claim 7, wherein, in the general formula (19), A is a group represented by the formula (2), at least one K¹ is a group represented by the formula (5) with the proviso that X¹ and X² are each an oxygen atom and that k is 0, and at least one K² is a group represented by the formula (7) with the proviso that X² is an oxygen atom and that l is 0.

9. The polymerizable compound as recited in claim 4, wherein, in the general formula (19), A is a group represented by the formula (2), at least one K¹ is a group represented by the formula (5) with the proviso that k is 0, and Z is a terminal group having a thiolactone group or a thioester group.

10. The polymerizable compound as recited in claim 4, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that I is 0, and Z is a terminal group having a thiolactone group or a thioester group.

11. The polymerizable compound as recited in claim 10, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that I is 0, and Z is a terminal group having a thiolactone group or a thioester group.

12. The polymerizable compound as recited in claim 4, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that l is 0, L is a group represented by the formula (10) or (11), and Z is a terminal group having a thiolactone group or a thioester group.

13. The polymerizable compound as recited in claim 12, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that i is 0, L is a group represented by the formula (10), and Z is a terminal group having a thiolactone group or a thioester group.

14. The polymerizable compound as recited in claim 4, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that I is 0, and Z is a terminal group having a lactone group or an ester group.

15. The polymerizable compound as recited in claim 4, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that I is 0, L is a group represented by the formula (10) or (11), and Z is a terminal group having a lactone group or an ester group.

16. The polymerizable compound as recited in claim 15, wherein, in the general formula (19), A is a group represented by the formula (2), at least one K¹ is a group represented by the formula (5) with the proviso that X¹ and X² are each an oxygen atom and that k is 0, at least one K² is a group represented by the formula (7) with the proviso that X² is an oxygen atom and that I is 0, L is a group represented by the formula (10), and Z is a terminal group having a lactone group or an ester group.

17. A process for producing a polymerizable compound represented by the general formula (19a) given below and having an alicyclic structure and a terminal group, comprising reacting a compound represented by the general formula (20) given below with a compound represented by the general formula (21) given below: 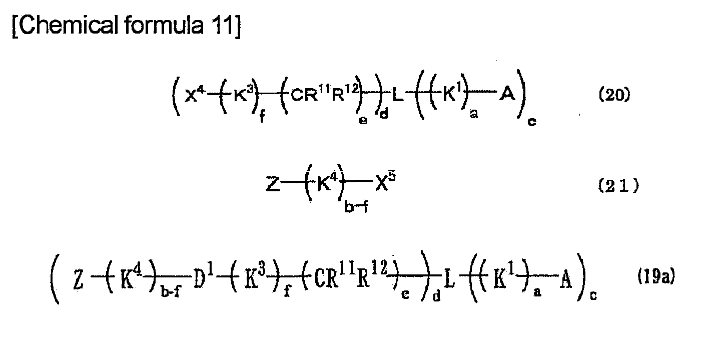 wherein
X⁴ represents a group selected from a hydrogen atom, a halogen atom, an alkylsulfonyloxy group, a perfluoroalkylsulfonyloxy group and an alkyl-substituted phenylsulfonyloxy group, in which case X⁵ represents a group selected from a hydrogen atom, a hydroxyl group, a mercapto group and an NH₂ group, or X⁴ represents a group selected from a hydrogen atom, a hydroxyl group, a mercapto group and an NH₂ group, in which case X⁵ represents a group selected from a hydrogen atom, a halogen atom, an alkylsulfonyloxy group, a perfluoroalkylsulfonyloxy group and an alkyl-substituted phenylsulfonyloxy group;
D¹ represents a linking group formed by the reaction between X⁴ and X⁵;
K³ and K⁴ each represent a linking group;
A, K¹, L, Z, a, b, c and d are the same as defined above; and
e is an integer of 0 to 10 and f is an integer of 0 or more.

18. A process for producing a polymerizable compound represented by the general formula (19b) given below and having an alicyclic structure and a terminal group, comprising reacting a compound represented by the general formula (22) given below with a compound represented by the general formula (23) given below: 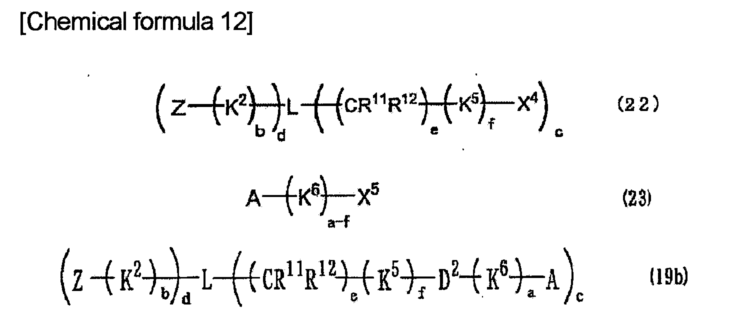 wherein
X⁴ represents a group selected from a hydrogen atom, a halogen atom, an alkylsulfonyloxy group, a perfluoroalkylsulfonyloxy group and an alkyl-substituted phenylsulfonyloxy group, in which case X⁵ represents a group selected from a hydrogen atom, a hydroxyl group, a mercapto group and an NH₂ group, or X⁴ represents a group selected from a hydrogen atom, a hydroxyl group, a mercapto group and an NH₂ group, in which case X⁵ represents a group selected from a hydrogen atom, a halogen atom, an alkylsulfonyloxy group, a perfluoroalkylsulfonyloxy group and an alkyl-substituted phenylsulfonyloxy group;
D² represents a linking group formed by the reaction between X⁴ and X⁵;
K⁵ and K⁶ each represent a linking group;
A, K², L, Z, a, b, c and d are the same as defined above; and
e is an integer of 0 to 10 and f is an integer of 0 or more.

19. A polymer comprising, as a constituting component, a polymerizable compound having an alicyclic structure and a polymerizable group and represented by the general formula (1).

20. A photoresist composition comprising a polymer comprising, as a constituting component, a polymerizable compound having an alicyclic structure and a polymerizable group and represented by the general formula (1).
